(19) Europäisches Patentamt
European Patent Office
Office européen des brevets

(11) Numéro de publication : 0 213 984 B1

(12) # FASCICULE DE BREVET EUROPEEN

(45) Date de publication du fascicule du brevet : 09.01.91 Bulletin 91/02

(51) Int. Cl.[5] : C07D 209/08, C07D 209/34, A61K 31/40

(21) Numéro de dépôt : 86401528.4

(22) Date de dépôt : 09.07.86

(54) **Nouveaux dérivés de l'indole carboxamide, leurs sels, procédé et intermédiaires de préparation, application à titre de médicaments et compositions les renfermant.**

(30) Priorité : 11.07.85 FR 8510648

(43) Date de publication de la demande : 11.03.87 Bulletin 87/11

(45) Mention de la délivrance du brevet : 09.01.91 Bulletin 91/02

(84) Etats contractants désignés : AT BE CH DE FR GB IT LI LU NL SE

(56) Documents cités : EP-A- 0 099 766

(73) Titulaire : ROUSSEL-UCLAF
35, boulevard des Invalides
F-75007 Paris (FR)

(72) Inventeur : Clemence, François
2, rue Turgot
F-75009 Paris (FR)
Inventeur : Guillaume, Jacques
62, rue Pexerecourt
F-75020 Paris (FR)
Inventeur : Hamon, Gilles
40, rue de Bagneux
F-92120 Montrouge (FR)

(74) Mandataire : Petranker, Léon et al
Département des Brevets ROUSSEL UCLAF
B.P. no 9 111, route de Noisy
F-93230 Romainville (FR)

EP 0 213 984 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne des dérivés de l'indole carboxamide, ainsi que leurs sels, le procédé et des intermédiaires de préparation, l'application à titre de médicaments de ces dérivés, les compositions les renfermant. EP-A-009976 décrit des dérivés de la 1,3-dihydro 4-(1-hydroxy 2-aminoéthyl) 2H-indol-2-one possédant des activités anti-hypertensives, anti-inflammatoires et antiulcéreuses.

L'invention a pour objet des dérivés de l'indole carboxamide, ainsi que leurs sels d'addition avec les acides minéraux ou organiques, caractérisés en ce qu'ils répondent à la formule générale (I) :

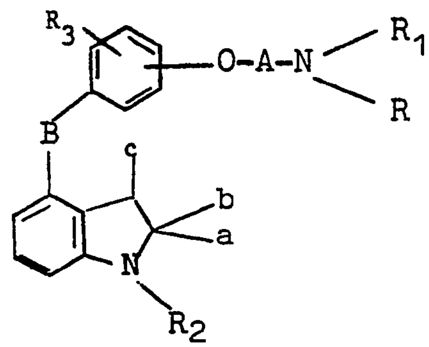

I

dans laquelle R et $R_1$ représentent, indépendamment l'un de l'autre, un atome d'hydrogène, un radical alkyle linéaire renfermant de 1 à 5 atomes de carbone, un radical alkyle ramifié renfermant de 3 à 5 atomes de carbone, un radical cycloalkyle renfermant de 3 à 7 atomes de carbone, un radical cycloalkylalkyle renfermant de 4 à 7 atomes de carbone, ou un radical aralkyle renfermant de 7 à 12 atomes de carbone éventuellement substitué par 1, 2 ou 3 radicaux choisis dans le groupe constitué par les halogènes et les radicaux méthyle, éthyle, méthoxy, éthoxy, trifluoro méthyle, méthylthio, amino et nitro, ou R et $R_1$ forment ensemble un hétérocycle saturé ou insaturé pouvant renfermer un second hétéroatome choisi parmi les atomes d'oxygène, de soufre et d'azote, cet atome d'azote étant éventuellement substitué par un radical alkyle renfermant de 1 à 5 atomes de carbone, par un radical phényle, phényle substitué ou naphtyle ou par un radical aralkyle renfermant de 7 à 12 atomes de carbone, $R_3$ représente un atome d'hydrogène, un radical alkyle renfermant de 1 à 5 atomes de carbone, un radical alkoxy renfermant de 1 à 3 atomes de carbone, un atome de chlore, de brome ou d'iode, un radical nitro ou un radical amino éventuellement substitué par un groupe acyle aliphatique renfermant de 2 à 5 atomes de carbone ou un radical alkyle renfermant de 1 à 5 atomes de carbone, a représente ensemble avec b une fonction oxo, ou représente ensemble avec c une liaison carbone-carbone, b représente un atome d'hydrogène, ou ensemble avec a une fonction oxo, c représente un atome d'hydrogène, ou ensemble avec a représente une liaison carbone-carbone, A représente une chaîne –(CH2)n– dans laquelle n peut prendre les valeurs 2, 3, 4 ou 5, ou une chaîne

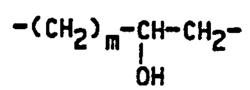

dans laquelle m peut prendre les valeurs 1, 2 ou 3, B représente une chaîne –CO–NH– ou –NH–CO–, $R_2$ représente un atome d'hydrogène, un radical alkyle linéaire renfermant de 1 à 5 atomes de carbone ou un radical alkyle ramifié renfermant de 3 à 5 atomes de carbone.

Dans la formule générale (I) et dans ce qui suit, le terme radical alkyle linéaire renfermant de 1 à 5 atomes de carbone désigne, de préférence, un radical méthyle, éthyle ou propyle ; le terme radical alkyle ramifié renfermant de 3 à 5 atomes de carbone désigne, de préférence, un radical isopropyle ou tert-butyle, le terme cycloalkyle renfermant de 3 à 7 atomes de carbone désigne, de préférence, un radical cyclopentyle; le terme radical cycloalkylalkyle renfermant de 4 à 7 atomes de carbone désigne, de préférence, un radical cyclopropylméthyle ; le terme radical aralkyle renfermant de 7 à 12 atomes de carbone désigne, de préférence, un radical benzyle ou phénéthyle, éventuellement substitué par 1, 2 ou 3 radicaux choisis parmi le groupe des halogènes, méthyle, éthyle, méthoxy, éthoxy, trifluorométhyle, méthylthio, amino et nitro ; lorsque R et $R_1$ forment ensemble, avec l'atome d'azote, un hétérocycle, il s'agit, par exemple, d'un cycle pyrrolidino, pipéridino, morpholino, pipérazinyle, méthylpipérazinyle, éthyl pipérazinyle ou propyl pipérazinyle ; le phényle substitué peut porter les mêmes substituants que les radicaux benzyle et phénéthyle ci-dessus.

Le terme radical alkoxy renfermant de 1 à 3 atomes de carbone désigne le radical méthoxy, éthoxy ou

propoxy. Le terme acyle aliphatique renfermant de 2 à 5 atomes de carbone désigne de préférence le radical acétyle ou propionyle.

Les sels d'addition avec les acides minéraux ou organiques peuvent être, par exemple, les sels formés avec les acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcane sulfoniques tels que les acides méthane et éthane sulfoniques, arylsulfoniques, tels que les acides benzène et paratoluène sulfoniques et arylcarboxyliques.

Parmi les produits objet de l'invention, on peut citer notamment les dérivés répondant à la formule (I) ci-dessus, ainsi que leurs sels d'addition avec les acides minéraux ou organiques, caractérisés en ce que, dans ladite formule (I), $R_2$ représente un atome d'hydrogène.

Parmi ceux-ci, on peut citer plus particulièrement les dérivés caractérisés en ce que dans ladite formule (I), a et c forment ensemble une liaison carbone-carbone.

Parmi les produits, objet de l'invention, on peut encore citer les dérivés répondant à la formule (I) ci-dessus ainsi que leurs sels d'addition avec les acides minéraux ou organiques caractérisés en ce que dans la formule (I) $R_3$ représente un atome d'hydrogène.

Parmi les produits, objet de l'invention, on peut encore citer les dérivés répondant à la formule (I) ci-dessus ainsi que leurs sels d'addition avec les acides minéraux ou organiques caractérisés en ce que dans la formule (I) la chaîne latérale

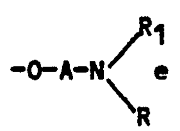

est fixée en position ortho.

Parmi les produits, objet de l'invention, on peut encore citer les dérivés répondant à la formule (I) ci-dessus ainsi que leurs sels d'addition avec les acides minéraux ou organiques caractérisés en ce que dans la formule (I) B représente une chaîne –NH–CO, NH étant du côté de l'indole.

Parmi les produits préférés ci-dessus, on peut citer tout particulièrement :

– le 2-/2-/(1,1-diméthyléthyl) amino/éthoxy/N-(1H-indol-4-yl) benzamide,
– le 2-/3-/(1,1-diméthyléthyl) amino/2-hydroxy propoxy/N-(1H-indol-4-yl) benzamide,
– le 2-/3-//bis-(1-méthyléthyl)/amino/2-hydroxypropoxy/N-(1H-indol-4-yl) benzamide,
– le 2-/3-/(1,1-diméthyléthyl) amino/propoxy/N-(1H-indol-4-yl) benzamide,

ainsi que leurs sels d'addition avec les acides minéraux ou organiques.

L'invention a également pour objet un procédé de préparation des dérivés tels que définis par la formule I ci-dessus, ainsi que de leurs sels, caractérisé en ce que l'on fait réagir un dérivé de formule II :

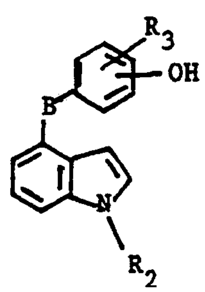

II

dans laquelle B, $R_2$ et $R_3$ ont la signification déjà indiquée, avec un halogénure de formule III :

Hal-G III

dans laquelle Hal représente un atome de chlore, de brome ou d'iode et G représente un groupement de formule $-(CH_2)_n-D$, dans laquelle D représente un atome de chlore, de brome, d'iode, un radical hydroxy ou un sulfonate de ce radical hydroxy, et n a la signification déjà indiquée, ou G représente un groupement

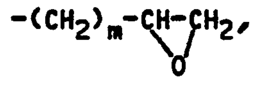

dans laquelle m a la signification déjà indiquée, pour obtenir un dérivé de formule IV :

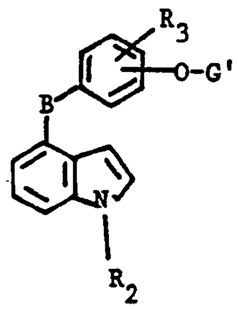

IV

dans laquelle B, $R_2$ et $R_3$ ont la signification déjà indiquée, et G′ représente un groupement de formule $-(CH_2)_n-Hal$ dans laquelle n et Hal ont la signification déjà indiquée ou un groupement de formule

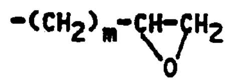

défini ci-dessus, que l'on fait réagir avec une amine de formule V :

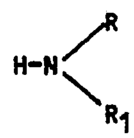

V

dans laquelle R et $R_1$ ont la signification déjà indiquée, pour obtenir un produit de formule IA :

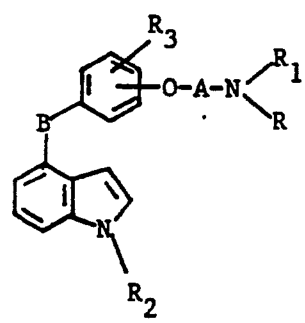

$I_A$

dans laquelle A, B, R, $R_1$, $R_2$ et $R_3$ ont la signification déjà indiquée, que soit l'on isole et, si désiré, salifie, ou si désiré, soumet à un agent d'alkylation, l'amine secondaire de la chaîne latérale lorsque R ou $R_1$ représente un atome d'hydrogène, soit soumet à l'action d'un agent d'halogénation pour obtenir un produit de formule VI :

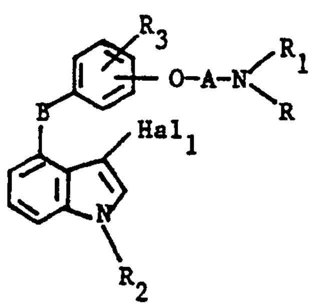

VI

dans laquelle $Hal_1$ représente un atome de brome ou de chlore, et A, B, R, $R_1$, $R_2$ et $R_3$ ont la signification déjà indiquée, que l'on soumet à une hydrolyse pour obtenir un produit de formule $I_B$ :

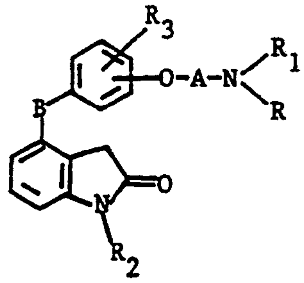

$I_B$

dans laquelle A, B, R, $R_1$, $R_2$ et $R_3$ ont la signification déjà indiquée, que l'on isole et, si désiré, salifie.

Lorsque l'on veut obtenir une chaîne de formule $-(CH_2)_n-$ pour A, si l'on utilise un halogénure de formule III répondant à la formule :

$$Hal-(CH_2)_n-D$$

dans laquelle D représente un atome de chlore, de brome, ou d'iode et Hal a la signification déjà indiquée, il est préférable que D et Hal représente deux halogènes différents pour éviter la condensation de deux molécules de dérivé de formule IV. C'est ainsi que, par exemple, pour D représentant un atome de chlore, on choisira pour Hal un halogénure plus réactif tel que le bromure.

Lorsque l'on utilise un halogénure hydroxylé de formule III répondant à la formule :

$$HO-(Ch_2)_n-Hal$$

dans laquelle n et Hal ont la signification déjà indiquée, on opère de préférence en présence de triphényl phosphine et d'azodicarboxylate d'éthyle dans le tétrahydrofuranne.

Avantageusement, on utilise un sulfonate de ce dérivé hydroxylé ; dans ce cas, de préférence, on utilise son tosylate de formule :

$$TsO-(CH_2)_n-Hal$$

dans laquelle Ts représente un radical tosyle (4-méthylbenzène sulfonate) et n et Hal ont la signification déjà indiquée.

On opère alors par transfert de phase en utilisant comme phase aqueuse de préférence une solution aqueuse d'un hydroxyde alcalin tel que l'hydroxyde de potassium ou de sodium, et comme phase organique non miscible à l'eau un solvant tel que le benzène, en présence d'un agent de transfert tel qu'un sel d'ammonium quaternaire de tétrabutyl ammonium, notamment le bromure ou l'hydrogénosulfate.

La réaction du produit de formule IV avec l'amine de formule V est réalisée par exemple au sein d'un solvant organique inerte tel que le dioxanne, le benzène, le toluène, le diméthylformamide, ou encore un alcool, de préférence l'éthanol, de préférence en présence d'un agent de condensation tel qu'un carbonate ou bicarbonate alcalin comme le carbonate de potassium, un hydroxyde alcalin comme la soude ou la potasse, ou une amine tertiaire comme la triéthylamine. On peut opérer également en utilisant comme solvant directement l'amine de formule V.

Lorsque l'on veut obtenir une chaîne de formule

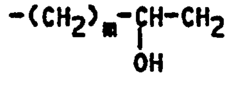

pour A, on utilise un halogénure de formule

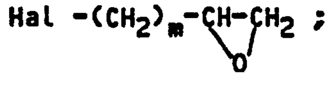

dans ce cas, Hal représente de préférence un atome de chlore. La réaction du dérivé de formule II avec l'halogénure de formule III est alors réalisée de préférence en présence d'une base telle que le carbonate de potassium ou de sodium, la soude ou la potasse.

La réaction du dérivé de formule IV dans laquelle G' représente une chaîne

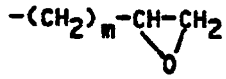

avec l'amine de formule V est réalisée soit directement en utilisant l'amine comme solvant, soit en utilisant un solvant tel qu'un alcool aliphatique comme le méthanol ou l'éthanol.

Dans le produit de formule $I_A$, l'alkylation éventuelle de l'amine secondaire de la chaîne latérale est effectuée par action d'un halogénure d'alkyle en présence d'un carbonate alcalin tel que le carbonate de sodium ou de potassium, au sein d'un solvant organique.

Lorsque l'on veut effectuer une méthylation, on utilise de préférence le formaldéhyde en présence d'un agent réducteur tel que le cyanoborohydrure de sodium au sein d'un solvant tel qu'un alcool aliphatique comme le méthanol.

On peut également faire réagir le paratoluène sulfonate de méthyle en présence d'un carbonate alcalin tel que le carbonate de potassium ou de sodium au sein d'un solvant organique tel que le xylène.

L'halogénation des dérivés de formules $I_A$ peut être utilisée, par exemple, à l'aide du complexe bromé de la pyridine de formule :

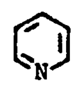 , $Br_2$, HBr

dans le cas de la bromation. Elle est réalisée avantageusement à l'aide d'un N-halo succinimide, de préférence le N-bromo ou le N-chloro succinimide : on opère dans le dioxanne ou de préférence dans l'acide acétique. Le produit de formule (VI) obtenu est de préférence un produit chloré.

L'hydrolyse du produit de formule (VI) est réalisée, de préférence à l'aide d'un acide minéral tel que l'acide phosphorique, l'acide sulfurique, ou de préférence l'acide chlorhydrique en solution aqueuse. Cette solution peut être utilisée concentrée, mais de préférence diluée par exemple en solution normale. On peut utiliser, en outre, un solvant tel qu'un alcool aliphatique comme l'éthanol.

Les dérivés de formule II peuvent être préparés comme suit :

Pour obtenir un dérivé de formule $II_A$ :

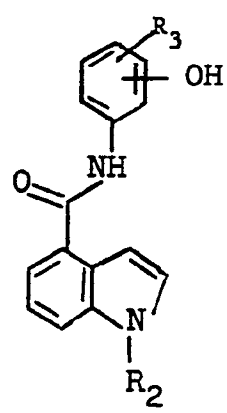

$II_A$

dans laquelle $R_2$ et $R_3$ ont la signification déjà indiquée, l'on fait réagir l'indole 4-carboxylate de méthyle ou d'éthyle correspondant ou l'acide indol-4-carboxylique correspondant avec un dérivé d'aminophénol de formule VII :

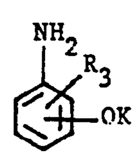

VII

dans laquelle K et $R_3$ représentent un atome d'hydrogène ou un groupe protecteur du radical hydroxy pour

obtenir un dérivé de formule VIII :

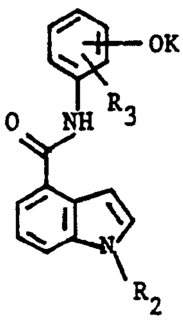

VIII

dans laquelle K, $R_2$ et $R_3$ ont la signification déjà indiquée, dont on libère le cas échéant la fonction hydroxy pour obtenir le produit de formule $II_A$ cherché.

Par groupe protecteur K du radical hydroxy, on entend par exemple le radical benzyle ou tosyle mais de préférence le radical tosyle.

La réaction de l'indole 4-carboxylate de méthyle ou d'éthyle avec le dérivé de formule VII est réalisée de préférence en présence de triisobutylaluminium. Le solvant utilisé est de préférence le chloroforme. On opère avantageusement au reflux du mélange réactionnel.

La réaction de l'acide indol-4-carboxylique avec le dérivé de formule VII est réalisée en présence d'un agent déshydratant tel que le carbonyldiimidazole ou de préférence le dicyclohexylcarbodiimide, au sein d'un solvant tel que le tétrahydrofuranne.

Le déblocage de la fonction hydroxy du dérivé de formule VIII est réalisé par hydrogénolyse lorsque K représente un radical benzyle ou par saponification lorsque K représente un radical tosyle de préférence à l'aide de soude ou de potasse, dans un solvant tel qu'un alcanol de faible poids moléculaire tel que le méthanol ou, de préférence, l'éthanol.

Pour obtenir un dérivé de formule $II_B$ :

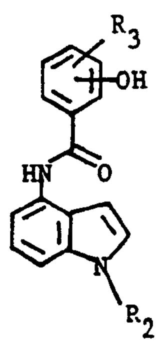

$II_B$

dans laquelle $R_2$ et $R_3$ ont la signification déjà indiquée, l'on fait réagir le 4-amino indole correspondant avec un carboxylate de méthyle ou d'éthyle du phénol ou avec un acide hydroxy benzoïque pour obtenir le dérivé de formule $II_B$ cherché.

Lorsque l'on utilise un carboxylate de méthyle ou d'éthyle du phénol, on opère, de préférence, en présence de triisobutylaluminium dans un solvant tel que le chloroforme. On opère avantageusement au reflux du mélange réactionnel.

Lorsque l'on utilise un carboxylate de méthyle ou d'éthyle du phénol, on opère dans des conditions identiques à celles indiquées pour la préparation du produit $II_A$ au départ de l'indole 4-carboxylate de méthyle ou d'éthyle.

Lorsque l'on utilise l'acide hydroxy benzoïque, on opère dans des conditions identiques à celles indiquées pour la préparation du produit $II_A$ au départ de l'acide indol-4-carboxylique.

Dans une variante du procédé décrit ci-dessus pour la préparation des dérivés de formule I, dans laquelle A représente une chaîne $-(CH_2)_n-$, on fait réagir un dérivé de formule II avec un dérivé de formule IX :

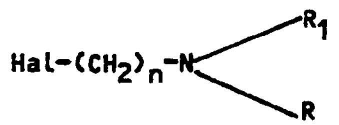 IX

dans laquelle Hal, n, R et $R_1$ ont la signification déjà indiquée, pour obtenir le dérivé de formule $I_A$ recherché que l'on transforme si désiré un produit de formule $I_B$ correspondant comme indiqué ci-dessus. On fait réagir le dérivé de formule IX sous forme d'anine libre ou, de préférence sous forme de sel comme un chlorhydrate.

Selon une autre variante du procédé décrit ci-dessus pour la préparation d'un dérivé de formule (I), dans laquelle $R_3$ représente un radical amino éventuellement substitué, on réduit le dérivé dans lequel $R_3$ représente un radical nitro et le cas échéant, on fait réagir le dérivé amino avec un dérivé réactif du substituant que l'on désire obtenir. Les conditions opératoires correspondantes sont connues de l'homme de métier.

Les dérivés de formule (I) présentent un caractère basique. On peut avantageusement préparer les sels d'addition des dérivés de formule (I), en faisant réagir, en proportions sensiblement stoechiométriques, un acide minéral ou organique avec ledit dérivé de formule (I). Les sels peuvent être préparés sans isoler les bases correspondantes.

Les dérivés, objet de la présente invention, possèdent de très intéressantes propriétés pharmacologiques ; ils sont doués notamment de remarquables propriétés antiarythmiques.

Ces propriétés sont illustrées plus loin dans la partie expérimentale.

Ces propriétés justifient l'utilisation des dérivés de l'indole carboxamide de formule I, ainsi que de leurs sels, à titre de médicaments.

La présente demande a ainsi également pour objet l'application, à titre de médicaments, des dérivés de l'indole carboxamide, tels que définis par la formule (I), ainsi que de leurs sels d'addition avec les acides pharmaceutiquement acceptables.

Parmi les médicaments, objet de l'invention, on retient, de préférence, les médicaments caractérisés en ce qu'ils sont constitués par les nouveaux dérivés de l'indole carboxamide répondant à la formule (I) dans laquelle $R_2$ représente un atome d'hydrogène ainsi que par leurs sels d'addition avec les acides pharmaceutiquement acceptables.

Parmi ceux-ci, on retient notamment ceux répondant à la formule (I), dans laquelle a et c forment ensemble une liaison carbone-carbone ainsi que leurs sels d'addition avec les acides pharmaceutiquement acceptables.

Parmi les médicaments selon l'invention, on peut encore citer ceux caractérisés en ce qu'ils sont constitués par les nouveaux dérivés de formule (I) dans laquelle $R_3$ représente un atome d'hydrogène ainsi que par leurs sels d'addition avec les acides pharmaceutiquement acceptables.

Parmi les médicaments selon l'invention, on peut encore citer ceux caractérisés en ce qu'ils sont constitués par les nouveaux dérivés de formule (I) dans laquelle la chaîne latérale

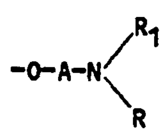

est fixée en position ortho ainsi que par leurs sels d'addition avec les acides pharmaceutiquement acceptables.

Parmi les médicaments selon l'invention, on peut également citer ceux caractérisés en ce qu'ils sont constitués par les nouveaux dérivés de formule (I) dans laquelle B représente une chaîne –N–H–CO, NH étant du côté de l'indole, ainsi que par leurs sels d'addition avec les acides pharmaceutiquement acceptables.

Parmi les différents médicaments préférés ci-dessus, on retient tout particulièrement ceux constitués par les dérivés dont les noms suivent :

– le 2-/2-/(1,1-diméthyléthyl) amino/éthoxy/N-(1H-indol-4-yl) benzamide,
– le 2-/3-/(1,1-diméthyléthyl) amino/2-hydroxy propoxy/N-(1H-indol-4-yl) benzamide,
– le 2-/3-//bis-(1-méthyléthyl)/amino/2-hydroxypropoxy/N-(1H-indol-4yl) benzamide,
– le 2-/3-/(1,1-diméthyléthyl) amino/propoxy/N-(1H-indol-4-yl) benzamide,

ainsi que par leurs sels d'addition avec les acides pharmaceutiquement acceptables.

Les médicaments selon l'invention trouvent leur emploi, par exemple dans le traitement des arythmies.

La dose usuelle, variable selon le dérivé utilisé, le sujet et l'affection en cause peut être par exemple, de 50 mg à 1 g par jour. Par voie orale, chez l'homme, le dérivé de l'exemple 5 peut être administré à la dose quotidienne de 200 mg à 800 mg, par exemple pour le traitement des arythmies ventriculaires, supraventriculaires et jonctionnelles, soit environ de 3 mg à 12 mg par kilogramme de poids corporel.

L'invention a également pour objet les compositions pharmaceutiques qui renferment au moins un dérivé précité ou l'un de ses sels d'addition avec les acides pharmaceutiquement acceptables, à titre de principe actif.

A titre de médicaments, les dérivés répondant à la formule (I) et leurs sels d'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple, les comprimés, simples ou dragéifiés, les gélules, les granulés, les suppositoires, les préparations injectables ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention s'étend en outre aux produits nouveaux répondant aux formules (II), (IV) et (VI) :

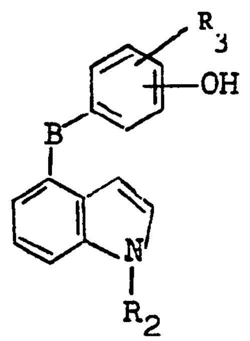

II

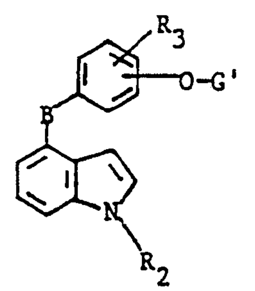

IV

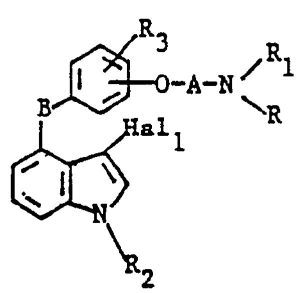

VI

dans laquelle B, R, $R_1$, $R_2$, $R_3$, G' et $Hal_1$ ont la signification déjà indiquée.

En plus des produits décrits dans les exemples, les produits suivants constituent des produits nouveaux pouvant être obtenus dans le cadre de la présente invention :

- le 2-/3-/(1,1-diméthylpropyl) amino/2-hydroxy propoxy/N-(1H-indol-4-yl) benzamide,
- le 2-/3-/(1,1-diméthyléthyl) amino/2-hydroxy propoxy/N-(1-méthyl 1H-indol-4-yl) benzamide,
- le 2-/3-(4-morpholinyl) 2-hydroxy propoxy/N-(1H-indol-4-yl) benzamide.

Les exemples qui suivent illustrent la présente invention.

**Exemple n° 1 : N-/2-/3-/(1,1-diméthyléthyl) amino/ 2-hydroxy propoxy/ phényl/1H-indol-4-carboxamide et son oxalate neutre.**

Stade A : N-/2-/(2-oxiranyl) méthoxy/phényl/1H-indol-4-carboxamide.

On chauffe au reflux pendant 24 heures, sous atmosphère inerte, une solution de 3 g de N-(2-hydroxy-phényl) 1H-indol-4-carboxamide et 1,65 g de carbonate de potassium dans 100 $cm^3$ d'acétone avec 4,7 $cm^3$ d'épichlorhydrine, purifie par chromatographie sur silice (éluant : acétate d'éthyle-triéthylamine : 9-1), amène à sec, empâte dans le pentane, filtre, sèche sous pression réduite à 60°C et obtient 2,8 g du produit attendu F°≃122°C.

Stade B : N-/2-/3-/(1,1-diméthyléthyl) amino/2-hydroxy propoxy/phényl/1H-indol-4-carboxamide et son oxalate neutre

On chauffe à 80°C sous agitation et atmosphère inerte pendant 1 heure une solution de 2,8 g de produit du stade A dans 40 $cm^3$ d'éthanol, avec 7,6 $cm^3$ de ter butylamine, purifie par chromatographie sur silice (éluants : acétate d'éthyle-triéthylamine 9-1, puis chloroforme-méthanol 5-5) et obtient 3,05 g de la base du produit attendu (poudre amorphe beige).

Formation de l'oxalate :

On dissout 2,9 g de base ci-dessus dans 300 $cm^3$ d'acétone, ajoute 480 mg d'acide oxalique, porte pendant 15 mn au reflux, concentre à environ 200 $cm^3$, glace, filtre, sèche sous pression réduite à 80°C et obtient 2,9 g du produit attendu ; 2,35 g après recristallisation dans le méthanol F°≃200°C.

Préparation de N-(2-hydroxyphényl) 1H-indol-4-carboxamide utilisé au départ de l'exemple 1 :

STADE A : N-/2-/(4-méthylphényl) sulfonyloxy/phényl/1H-indol-4-carboxamide.

A une solution de 18 g de 4-méthylbenzènesulfonate de 2-aminophénol dans 300 $cm^3$ de chloroforme, on ajoute sous agitation et atmosphère inerte 140 $cm^3$ de triisobutylaluminium en solution dans le toluène à 1,1 mole/l, agite pendant 15 mn, ajoute une solution de 12,15 g d'indol-4-carboxylate de méthyle dans 120 $cm^3$ de chloroforme et porte au reflux pendant 20 heures. On refroidit à 0,–10°C et ajoute 500 $cm^3$ de solution aqueuse N d'acide chlorhydrique, agite pendant 15 minutes, purifie la phase chloroformique par chromatographie sur silice, (éluant : chlorure de méthylène), et obtient 25 g du produit attendu F°9=135°C.

STADE B : Préparation du N-(2-hydroxyphényl) 1H-indol-4-carboxamide.

On ajoute sous agitation et atmosphère inerte 250 $cm^3$ d'une solution de potasse dans l'éthanol à 10 g pour 100 $cm^3$ à une suspension de 25 g de N-/2-/(4-méthylphényl) sulfonyloxy/phényl/1H-indol-4-carboxamidedans 50 $cm^3$ d'éthanol à 95°C, laisse sous agitation pendant 21 heures, ajoute 1 litre d'eau glacée, acidifie à l'aide d'une solution aqueuse concentrée d'acide chlorhydrique, agite encore 15 mn, filtre, sèche, empâte au reflux dans 1,5 l de chlorure de méthylène, concentre à 300 $cm^3$ environ, filtre, sèche à 80°C sous pression réduite et obtient 13 g du produit attendu F°≃208°C.

Spectre UV (Ethanol) :

| | | | |
|---|---|---|---|
| max. | 230 nm | $E^1_1$ = 1 012 | ε = 25 500 |
| infl. | 265 nm | $E^1_1$ = 208 | |
| max. | 304 nm | $E^1_1$ = 564 | ε = 14 200 |
| infl. | 318 nm | $E^1_1$ = 482 | |

**Exemple 2 : N-/2-/2-/(1,1-diméthyléthyl) amino/éthoxy/phényl/1H-indol-4-carboxamide et son chlorhydrate.**

**Stade A :** N-/2-(2-chloroéthoxy) phényl/1H-indol-4-carboxamide.

On chauffe à 60°C sous agitation et atmosphère inerte 2,5 g de 40 N-(2-hydroxyphényl) 1H-indol-4-carboxamide dans 100 $cm^3$ de benzène et 50 $cm^3$ d'acétonitrile avec 850 mg d'hydrogénosulfate de n-tétra-butylammonium, 3,6 $cm^3$ de 3-chloroéthylparatoluène sulfonate et 50 $cm^3$ de soude 5 N, refroidit, décante, extrait à l'acétate d'éthyle, purifie par chromatographie sur silice/éluant : dichloroéthane, amène à sec sous pression réduite à 50°C et obtient 1,65 g du produit attendu F°≃135°C.

**STADE B :** N-/2-/2-/(1,1-diméthyléthyl) amino/éthoxy/phényl/1H-indol-4-carboxamide et son chlorhydrate.

On chauffe à 120°C sous agitation et pression de 2 bars une solution de 2,3 g de produit du stade A dans 40 $cm^3$ d'éthanol et 37,5 $cm^3$ de ter butylamine pendant 24 heures, dilue par 400 $cm^3$ d'eau et 400 $cm^3$ d'acétate d'éthyle, alcalinise à l'aide de soude, sature de carbonate de potassium, extrait à l'acétate d'éthyle, empâte les cristaux obtenus à l'aide d'éther et obtient 2,3 g du produit attendu F°≃148°C.

Formation du chlorhydrate

On dissout la base ci-dessus dans 200 $cm^3$ d'acétate d'éthyle, ajoute jusqu'à pH acide une solution d'acétate d'éthyle chlorhydrique, chauffe au reflux pendant 15 mn, concentre à environ 100 $cm^3$, glace, filtre, sèche à 80°C sous pression réduite, recristallise dans l'isopropanol et obtient 2,15 g du produit attendu F°≃ 260°C. Spectre UV (éthanol)

| | | | |
|---|---|---|---|
| max | 229 nm | $E^1_1$ = 724 | ε = 28 100 |
| max | 302 nm | $E^1_1$ = 303 | ε = 11 750 |

**Exemple n° 3 : chlorhydrate de N-/2-/3-/(1,1-diméthyléthyl)amino/propoxy/phényl/1H-indol-4-carboxamide.**

**Stade A :** N-/2-(3-chloropropoxy) phényl/1H-indol-4-carboxamide.

On prépare sous agitation et atmosphère inerte une solution de 2 g de N-(2-hydroxyphényl) 1H-indol-4-carboxamide, 150 $cm^3$ de tétrahydrofuranne, 0,65 $cm^3$ de 3-chloropropanol et 2,1 g de triphénylphosphine, ajoute lentement 1,2 $cm^3$ d'azodicarboxylate d'éthyle, laisse pendant 3 heures sous agitation, ajoute 2,1 g de triphénylphosphine, 0,65 $cm^3$ de 3-chloropropanol puis, lentement, 1,2 $cm^3$ d'azodicarboxylate d'éthyle laisse à nouveau sous agitation pendant 16 heures, amène à sec, purifie par chromatographie sur silice (Eluant : chlorure de méthylène), amène à sec, empâte à l'éther isopropylique, filtre, sèche sous pression réduite et obtient 1,3 g du produit attendu F°≃144°C.

Stade B : chlorhydrate de N-/2-/3-/(1,1-diméthyléthyl) amino/ propoxy/phényl/1H-indol-4-carboxamide.

On chauffe à 100°C sous une pression de 2 bars et sous agitation 3,1 g de produit du stade A ci-dessus dans 60 $cm^3$ d'éthanol et 30 $cm^3$ de ter butylamine, dilue à l'aide de 200 $cm^3$ d'eau, acidifie à l'aide d'une solution aqueuse concentrée d'acide chlorydrique, filtre, lave à l'eau, empâte à l'éther, puis à chaud à l'aide d'acétone pendant 15 mn au reflux et obtient 2,9 g du produit attendu F°≃238°C.

Spectre UV (éthanol) :

max. 227 nm $E^1_1$ = 709 $\varepsilon$ = 28 400

infl. 262 nm $E^1_1$ = 130

max. 301 nm $E^1_1$ = 327 $\varepsilon$ = 13 100

infl. 320 nm $E^1_1$ = 227

**Exemple N° 4 : 2-/3-/(1,1-diméthyléthyl) amino/2-hydroxy propoxy/ N-(1H-indol-4-yl) benzamide et son oxalate neutre.**

En opérant selon un procédé analogue à celui décrit ci-dessus pour l'exemple 1, mais à partir de 3,5 g de 2-/N-(1H-indol-4-yl) amido/ phénol, on obtient 3 g de l'oxalate recherché F°≃190°C.

Spectre UV (Ethanol+Hcl 0,1 N) :

infl. 216 nm $E^1_1$ = 1 003

max. 296 nm $E^1_1$ = 264 $\varepsilon$ = 11 300

infl. 235, 274, 288, 309 nm.

Préparation du 2-/N-(1H-indol-4-yl) amido/phénol utilisé au départ de l'exemple n° 4.

On ajoute lentement sous agitation et atmosphère inerte 92 $cm^3$ d'une solution de triisobutylaluminium en solution à 1,1 mole/l dans le toluène, à une solution de 6,6 g de 4-amino indole dans 250 $cm^3$ de chloroforme, ajoute ensuite 9,6 $cm^3$ de salicylate de méthyle, porte au reflux pendant 20 heures, refroidit à température ambiante, ajoute 300 $cm^3$ d'acide chlorhydrique N et 300 $cm^3$ de chlorure de méthylène, lave à l'eau, sèche, amène à sec sous pression réduite à 50°C, empâte à l'éther, filtre, sèche à 60°C sous pression réduite et obtient 9,4 g du produit attendu. F°≃232°C.

Spectre UV-(Ethanol) :

| | | | |
|---|---|---|---|
| infl. | 216 nm | $E^1_1$ = 1 595 | |
| infl. | 233 nm | $E^1_1$ = 680 | $\varepsilon$ = 17 200 |
| infl. | 262 nm | $E^1_1$ = 187 | |
| infl. | 303 nm | $E^1_1$ = 482 | $\varepsilon$ = 12 200 |
| infl. | 314 nm | $E^1_1$ = 494 | $\varepsilon$ = 12 500 |

**Exemple N° 5 : chlorhydrate de 2-/2-/1,1-diméthyléthyl) amino/ éthoxy/N-(1H-indol-4-yl) benzamide.**

En opérant selon un procédé analogue à celui décrit ci-dessus pour l'exemple 2, mais à partir de 27 g de 2-/N-(1H-indol-4-yl) amido/ phénol benzamide, on obtient 4,4 g de chlorhydrate attendu F°≃248°C.

Spectre UV (éthanol ou éthanol+HCl 0,1 N) :

| | | | |
|---|---|---|---|
| infl. | 215 nm | $E^1_1$ = 1 099 | $\varepsilon$ = 42 600 |
| max. | 293 nm | $E^1_1$ = 285 | $\varepsilon$ = 11 100 |

**Exemple N° 6 : 2-/3-/(1,1-diméthyléthyl) amino/propoxy/N-(1H-indol-4-yl) benzamide et son oxalate acide.**

En opérant selon un procédé analogue à celui décrit ci-dessus pour l'exemple n° 3, mais à partir de 5 g de 2-/N-(1H-indol-4-yl) amido/ phénol benzamide, on obtient 3,1 g de la base attendue F°≃146°C, puis 3,1 g de son oxalate F°≃180°C.

Spectre UV de l'oxalate (Ethanol) :

| | | | |
|---|---|---|---|
| infl. | 218 nm | $E^1_1$ = 879 | |
| max. | 297 nm | $E^1_1$ = 246 | $\varepsilon$ = 11 200. |

**Exemple N° 7 : fumarate acide du N-/2-/2-(1-pipéridinyl) éthoxy/ phényl/1H-indol-4-carboxamide.**

On chauffe à 60°C pendant 3 heures sous agitation et atmosphère inerte 3 g de N-(2-hydroxyphényl) 1H-indol-4-carboxamide dans 50 cm³ de benzène, 25 cm³ d'acétonitrile, et 50 cm³ de soude aqueuse 5 N, avec 0,4 g d'hydrogénosulfate de N-tétrabutylammonium et 2,2 g de chlorhydrate de 2-pipéridino 1-chloroéthane, refroidit, décante, extrait à l'acétate d'éthyle, purifie par chromatographie sur silice (Eluant : acétate d'éthyle/triéthylamine 9-1) et obtient 3,6 g de la base attendue.

Formation du fumarate :

On dissout la base ci-dessus dans 100 cm³ d'isopropanol, ajoute 1,15 g d'acide fumarique, porte au reflux, glace, filtre, amène à sec sous pression réduite et obtient en 2 jets 3,80 g du produit attendu, F°≃

186°C après recristallisation dans l'éthanol.

Spectre UV :

| | | | | |
|---|---|---|---|---|
| infl. | 230 nm | $E^1_1$ = | 681 | |
| infl. | 260 nm | $E^1_1$ = | 141 | |
| max. | 300 nm | $E^1_1$ = | 271 | $\varepsilon$= 13 000 |
| infl. | 320 nm | $E^1_1$ = | 224 | |

**Exemple N° 8 : N-/2-/2-(diméthylamino) éthoxy/phényl/1H-indol-4carboxamide, et son tartrate acide.**

En opérant selon un procédé analogue à celui décrit ci-dessus pour l'exemple n°7, mais à partir de chlorhydrate de chlorure de diméthylaminoéthyle, on obtient 3,6 g de base F°≃110°C, puis 4,4 g de tartrate (point de ramolissement ≃110°C).

Spectre UV de tartrate (Ethanol) :

| | | | | |
|---|---|---|---|---|
| max. | 228 nm | $E^1_1$ = | 559 | $\varepsilon$= 26 500 |
| infl. | 263 nm | $E^1_1$ = | 123 | |
| max. | 300 nm | $E^1_1$ = | 261 | $\varepsilon$= 12 400. |

**Exemple N° 9 : N-/2-/2-/bis(1-méthyléthyl) amino/éthoxy/phényl/ 1H-indol-4-carboxamide et son chlorhydrate.**

En opérant selon un procédé analogue à celui décrit ci-dessus pour l'exemple n° 7, mais à partir de 3,5 g de chlorhydrate du chlorure de diisopropylaminoéthyle, on obtient 4 g de base F°≃145°C, puis 2,9 g de chlorhydrate F°≃214°C.

**Exemple N° 10 : N-(1H-indol-4-yl) 2-/2-(1-pipéridinyl)éthoxy/benzamide et son phosphate.**

On opère comme à l'exemple 7 à partir de 2,5 g de 2-/N-(1H-indol-4-yl) amido/phénol et 1,84 g de chlorhydrate de 2-pipéridino 1-chloroéthane. On obtient 3,0 g de produit attendu sous forme de base. F=154°C après recristallisation dans l'éther éthylique.

Formation du phosphate.

On dissout 2,7 g de la base ci-dessus dans 500 cm³ d'éthanol et ajoute 10 cm³ d'une solution 1M d'acide phosphorique dans l'éthanol. On chauffe au reflux, ajoute 500 cm³ de méthanol, filtre à chaud, concentre partiellement le milieu réactionnel, glace, filtre et sèche sous pression réduite à 80°C le produit obtenu. Après recristallisation dans un mélange éthanol-méthanol (1-1), on recueille 2,4 g de produit attendu. F = 238°C.

Analyse : $C_{22}H_{25}N_3O_2$, $H_3PO_4$ : 461,458.

| | | | | |
|---|---|---|---|---|
| Calculé : | C% 57,26 | H% 6,12 | N% 9,11 | P% 6,71 |
| Trouvé : | 57,1 | 6,2 | 9,0 | 6,6 |

**Exemple n° 11 : 2-/2-(diméthylamino) éthoxy/N-(1H-indol-4-yl) benzamide et son phosphate.**

On opère comme à l'exemple 7 à partir de 2,5 g de 2-/N-(1H-indol-4-yl) amido/phénol et 1,44 g de chlorhydrate de chlorure de diméthylaminoéthyle en maintenant le chauffage pendant 24 heures. On obtient 2,15 g de produit attendu sous forme de base. F=138°C après recristallisation dans l'éther éthylique.

Formation du phosphate.

On opère comme indique à l'exemple 10 à partir de 3,3 g de base préparée comme ci-dessus et en remplaçant l'éthanol par de l'isopropanol. On obtient 3,5 g de produit attendu.

Analyse : $C_{19}H_{21}N_3O_2$, $H_3PO_4$ : 421,37.

| | | | | |
|---|---|---|---|---|
| Calculé : | C% 54,15 | H% 5,70 | N% 9,97 | P% 7,35 |
| Trouvé : | 53,8 | 5,8 | 9,8 | 7,3 |

**Exemple n° 12 : 2-/2-/bis(1-méthyléthyl) amino/éthoxy/N-(1H-indol-4-yl) benzamide.**

On opère comme à l'exemple 7 à partir de 3,5 g de 2-/N-(1H-indol-4-yl) amido/phénol et de 2,8 g de chlorhydrate du chlorure de diisopropylamino éthyle. On obtient 5,7 g de produit brut que l'on purifie par chromatographie sur silice (éluant : chloroforme-acétone-triéthylamine 6-3-1) et recristallise dans le chloroforme, puis dans un mélange isopropanol-méthanol (2-1). On recueille 2,25 g de produit attendu. F=180°C.

Analyse : $C_{23}H_{29}N_3O_2$ : 379,48.

| | | | |
|---|---|---|---|
| Calculé : | C% 72,79 | H% 7,70 | N% 11,07 |
| Trouvé : | 72,9 | 7,9 | 10,9 |

**Exemple N° 13 : 2-/2-hydroxy 3-(propylamino) propoxy/N-(1H-indol-4-yl) benzamide et son oxalate acide.**

On chauffe 2 heures au reflux 3,1 g de 2-/(2-oxiranyl) méthoxy/N-(1Hindol-4-yl) benzamide préparé comme à l'exemple 4 dans 60 cm³ d'éthanol et 8,5 cm³ de n-propylamine. On élimine les solvants sous pression réduite à 50°C, chromatographie le résidu sur silice (éluant : chloroforme-méthanol 7-3) et recueille 2,8 g de produit attendu sous forme de base.

Formation de l'oxalate acide.

On dissout 1,7 g de base ci-dessus dans 200 cm³ d'isopropanol et 100 cm³ de méthanol au reflux et ajoute 583 mg d'acide oxalique. On concentre partiellement le milieu réactionnel, glace, filtre, et sèche sous pression réduite à 80°C le produit obtenu. On recueille 1,7 g de produit attendu. F=110°C.

Analyse : $C_{21}H_{25}N_3O_3$, $C_2H_2O_4$ : 467,487.

| | | | |
|---|---|---|---|
| Calculé : | C% 60,39 | H% 5,95 | N% 9,18 |
| Trouvé : | 60,2 | 6,0 | 9,2 |

**Exemple N° 14 : N-(1H-indol-4-yl) 2-/2-hydroxy 3-/(1-méthyléthyl)amino/propoxy/benzamide et son oxalate neutre.**

On opère comme à l'exemple 13 en remplaçant la n-propylamine par 8,6 cm³ d'isopropylamine. On obtient 2,8 g de produit attendu sous forme de base.

Formation de l'oxalate neutre.

On opère comme à l'exemple 13 à partir de 2,8 g de base préparée ci-dessus et 960 mg d'acide oxalique. On recueille 1,7 g de produit attendu. F=190°C.

Analyse : $C_{21}H_{25}N_3O_3$, 1/2 $C_2H_2O_4$ : 412,469.

| | | | |
|---|---|---|---|
| Calculé : | C% 64,06 | H% 6,35 | N% 10,19 |
| Trouvé : | 63,8 | 6,5 | 10,0 |

**Exemple N° 15 : 2-/3-//bis (1-méthyléthyl)/amino/2-hydroxy propoxy/N(1H-indol-4-yl) benzamide.**

On opère comme à l'exemple 13 en remplaçant la n-propylamine par 14 cm³ de diisopropylamine en maintenant le reflux pendant 5 heures. On obtient 4 g de produit brut que l'on chromatographie sur silice (éluant : chloroforme-acétate d'éthyle-triéthylamine 6-3-1) et recristallise dans l'acétate d'éthyle. On obtient 2,4 g de produit attendu. F=160°C.

Analyse : $C_{24}H_{31}N_3O_3$ : 409,533.

| | | | |
|---|---|---|---|
| Calculé : | C% 70,39 | H% 7,63 | N% 10,26 |
| Trouvé : | 70,4 | 7,8 | 10,2 |

**Exemple N° 16 : 2-/3-(diéthylamino) 2-hydroxy propoxy/N-(1H-indol-4-yl) benzamide et son chlorhydrate.**

On opère comme à l'exemple 13 en remplaçant la n-propylamine par 10 cm³ de diéthylamine. On obtient 3 g de produit attendu sous forme de base.

Formation du chlorhydrate.

On dissout 2,5 g de la base ci-dessus dans 65,6 cm³ d'acide chlorhydrique 0,1N et ajoute 10 cm³ de méthanol, chasse le méthanol sous pression réduite et lyophilise la solution. On récupère 2,67 g de produit attendu.

Analyse : $C_{22}H_{27}N_3O_3$, HCl : 417,939.

| | | | | |
|---|---|---|---|---|
| Calculé : | C% 63,23 | H% 6,75 | N% 10,05 | Cl% 8,48 |
| Trouvé : | 62,9 | 6,9 | 9,9 | 8,7 |

**Exemple N° 17 : Benzoate de 2-/3-/(1,1-diméthyléthyl) amino/2-hydroxy propoxy/N-(1H-indol-4-yl) benzamide.**

On dissout 2 g de la base obtenue à l'exemple 4 dans 200 cm³ d'isopropanol au reflux, et ajoute 640 mg d'acide benzoïque. On filtre à chaud la solution, concentre partiellement, glace, filtre et sèche sous pression réduite à 80°C le produit obtenu. Après recristallisation dans l'isopropanol, on recueille 2,0 g de produit attendu. F=190°C.

Analyse : $C_{22}H_{27}N_3O_3$, $C_7H_6O_2$ : 503,603.
Calculé : C% 69,17 H% 6,61 N% 8,34
Trouvé : 69,4 6,7 8,3

**Exemple N° 18 : 2-/3-/(1,1-diméthyléthyl) amino/2-hydroxy propoxy/N-2,3-dihydro 2-oxo 1H-indol-4-yl/benzamide et son oxalate neutre.**

**Stade A :** 2-/3-/(1,1-diméthyléthyl) amino/2-hydroxy propoxy/N-(3-chloro 1H-indol-4-yl) benzamide.

On agite pendant 1 heure à température ambiante et sous atmosphère inerte, le mélange comprenant 3,3 g de la base préparée à l'exemple 4, 40 cm³ d'acide acétique et 1,3 g de N-chloro succinimide. On dilue le milieu réactionnel avec de l'eau, alcalinise à l'aide d'ammoniaque et extrait à l'acétate d'éthyle. On élimine les solvants sous pression réduite, chromatographie le résidu sur silice (éluant : acétate d'éthyletriéthylamine 9-1) et obtient 2,5 g de produit attendu.

**Stade B** : 2-/3-/(1,1-diméthyléthyl) amino/2-hydroxy propoxy/N-2,3-dihydro 2-oxo 1H-indol-4-yl/benzamide et son oxalate neutre.

On chauffe 1 heure au reflux 2,5 g du produit obtenu ci-dessus dans 35 cm³ d'éthanol et 70 cm³ d'acide chlorhydrique 1N. On dilue à l'eau le milieu réactionnel, alcalinise avec de la soude et extrait à l'acétate d'éthyle. Après élimination du solvant sous pression réduite, on recueille 2,5 g de produit brut qui cristallise spontanément dans un mélange de solvants : chloroforme-acétate d'éthyle-triéthylamine (6-3-1). On obtient 1,9 g de produit attendu sous forme de base. F=160°C

Formation de l'oxalate neutre.

On dissout 1,9 g de la base ci-dessus dans 300 cm³ d'isopropanol et 100 cm³ de méthanol au reflux et ajoute 600 mg d'acide oxalique. On maintient le reflux pendant 15 minutes, refroidit, concentre partiellement le milieu réactionnel, glace, filtre et sèche sous pression réduite le produit brut attendu. Après recristallisation dans un mélange isopropanolméthanol (1-3), on obtient 1,8 g de l'oxalate attendu. F=234°C.

Analyse : $C_{22}H_{27}N_3O_4$, 1/2 $C_2H_2O_4$ : 442,495.
Calculé : C% 62,43 H% 6,38 N% 9,50
Trouvé : 62,2 6,4 9,3

**Exemple N° 19 : 2-/3-(4-méthyl 1-pipérazinyl) 2-hydroxy propoxy/N-(1H-indol-4-yl) benzamide et son oxalate acide.**

On chauffe au reflux pendant 1 heure 1,8 g de 2-/(2-oxiranyl) méthoxy/N-(1H-indol-4-yl) benzamide préparé comme indiqué à l'exemple 4 dans 36 cm³ d'éthanol et 6,5 cm³ de N-méthyl pipérazine. On élimine les solvants sous pression réduite, chromatographie le résidu sur silice (éluant : chloroforme-méthanol 9-1) et obtient 1,77 g de produit attendu sous forme de base.

Formation de l'oxalate acide.

On dissout 1,7 g de base ci-dessus dans 100 cm³ d'isopropanol, ajoute 524 mg d'acide oxalique, filtre et sèche sous pression réduite à 70°C le produit obtenu. Après recristallisation dans l'isopropanol, on recueille 0,96 g d'oxalate attendu. F≃130°C (décomposition).

Analyse : $C_{23}H_{28}N_4O_3$, $C_2H_2O_4$ : 498,54.
Calculé : C% 60,23 H% 6,07 N% 11,24
Trouvé : 60,3 6,3 11,2

**Exemple N° 20 : 2-/3-/4-(2 méthoxy phényl) 1-pipérazinyl/2-hydroxy propoxy/N-(1H-indol-4-yl) benzamide.**

On chauffe au reflux pendant 4 heures 1,6 g de 2-/(2-oxiranyl) méthoxy/N-(1H-indol-4-yl) benzamide dans 36 $cm^3$ d'éthanol avec 1,82 $cm^3$ de (méthoxy phényl) pipérazine. On élimine le solvant sous pression réduite, purifie le résidu par chromatographie sur silice (éluant : chloroforme-acétate d'éthyle-triéthylamine 6-3-1) et recueille 1,85 g de produit attendu.

Spectre UV (éthanol)

| | | | | |
|---|---|---|---|---|
| infl. | 235 nm | $E^1_1$ = | 449 | $\varepsilon$ = 22400 |
| max. | 287 nm | $E^1_1$ = | 222 | $\varepsilon$ = 11100 |
| infl. | 294 nm | $E^1_1$ = | 215 | |
| infl. | 310 nm | $E^1_1$ = | 179 | $\varepsilon$ = 8900 |

Spectre IR (chloroforme)

| | | |
|---|---|---|
| OH libre | : | 3600 $cm^{-1}$ |
| =C-NH | : | 3481 $cm^{-1}$-3373 $cm^{-1}$ |
| >C=O | : | 1661 $cm^{-1}$ |
| C=C + aromatique + amide II | | 1623 $cm^{-1}$-1600 $cm^{-1}$-1587 $cm^{-1}$-1538 $cm^{-1}$-1500 $cm^{-1}$-1485 $cm^{-1}$. |

**Exemple N° 21 : 2-/3-//2-(3,4-diméthoxy phényl) éthyl/amino/2-hydroxy propoxy/N-(1H-indol-4-yl) benzamide.**

On chauffe 1 heure au reflux 4 g de 2-/(2-oxiranyl) méthoxy/N-(1H-indol-4-yl) benzamide dans 48 $cm^3$ d'éthanol avec 6,57 $cm^3$ de diméthoxy phényl éthylamine, élimine le solvant sous pression réduite et purifie le résidu par chromatographie sur silice (éluant : chloroforme-méthanol 9-1). On obtient 4,60 g de produit attendu.

Spectre UV (éthanol)

| | | | | |
|---|---|---|---|---|
| infl. | 218 nm | $E^1_1$ = | 921 | |
| infl. | 230 nm | $E^1_1$ = | 528 | $\varepsilon$ = 25800 |
| max. | 288 nm | $E^1_1$ = | 222 | $\varepsilon$ = 10900 |
| max. | 299 nm | $E^1_1$ = | 211 | $\varepsilon$ = 10300 |
| infl. | 310 nm | $E^1_1$ = | 187 | $\varepsilon$ = 9150 |

Spectre IR (chloroforme)

amide secondaire NH : $3360 cm^{-1}$
C=O : $1659 cm^{-1}$
amide II : $1534 cm^{-1}$

aromatique : $1623 cm^{-1}$-$1601 cm^{-1}$-$1587 cm^{-1}$

aromatique 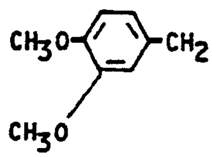 : $1516 cm^{-1}$

méthoxy : $2837 cm^{-1}$.

**Exemple N° 22 : 2-/3-(cyxlohexylamino) 2-hydroxy propoxy/N-(1H-indol-4-yl) benzamide.**

On chauffe 2 heures au reflux 2,5 g de 2-/(2-oxiranyl) méthoxy/N-(1H-indol-4-yl) benzamide dans 20 $cm^3$ d'éthanol avec 1,86 $cm^3$ de cyclohexylamine. On élimine le solvant sous pression réduite, chromatographie le résidu sur silice (éluant : chloroforme-méthanol 9-1) et obtient 1,97 g de produit attendu.

Spectre UV (éthanol)

| | | | | |
|---|---|---|---|---|
| infl. | 216 nm | $E^1_1$ = | 832 | |
| infl. | 234 nm | $E^1_1$ = | 320 | |
| infl. | 270 nm | $E^1_1$ = | 113 | |
| max. | 296 nm | $E^1_1$ = | 212 | $\varepsilon$ = 8650 |
| infl. | 304 nm | $E^1_1$ = | 199. | |

Spectre IR (chloroforme)

NH indole : $3480 cm^{-1}$
Amide : $1660 cm^{-1}$
Amide II : $1536 cm^{-1}$
Aromatiques : $1624 cm^{-1}$-$1600 cm^{-1}$-$1588 cm^{-1}$-$1503 cm^{-1}$-$1485 cm^{-1}$.

**Exemple N° 23 : 4-/3-/(1,1-diméthyléthyl) amino/2-hydroxy propoxy/N-(1H-indol-4-yl) benzamide et son fumarate neutre.**

Stade A : 4-/(2-oxiranyl) méthoxy/N-(1H-indol-4-yl) benzamide.

On opère comme à l'exemple 1 à partir de 5,37 g de 4-/N-(1H-indol-46yl) amido/phénol et 25,8 g $cm^3$ d'épichlorhydrine. Aprés chromatographie sur silice (éluant : chloroforme-acétate d'éthyle-triéthylamine 6-3-1) et empâtage dans l'éther isopropylique, on recueille 2,9 g de produit attendu. F=170°C.

**Stade B** : 4-/3-/(1,1-diméthyléthyl) amino/2-hydroxy propoxy/N-(1H-indol-4-yl) benzamide et son fumarate neutre.

On opère comme au stade B de l'exemple 1 à partir de 2,9 g de produit obtenu au stade A et obtient 3,5 g de produit attendu sous forme de base. F=212°C.

Formation du fumarate neutre.

On dissout 2,9 g de la base obtenue ci-dessus dans 300 cm³ d'isopropanol au reflux, ajoute 0,88 g d'acide fumarique puis 200 cm³ de méthanol et poursuit le chauffage au reflux pendant 30 minutes. On concentre partiellement le milieu réactionnel, glace, filtre, sèche sous pression réduite à 80°C et obtient 2,3 g de produit brut. Après recristallisation dans le mélange éthanol-méthanol-eau (10-10-1), on recueille 1,8 g de fumarate neutre. F>270°C.

Analyse : $C_{22}H_{27}N_3O_2$, 1/2 $C_4H_4O_4$ : 439,516.

| | | | |
|---|---|---|---|
| Calculé : | C% 65,59 | H% 6,65 | N% 9,56 |
| Trouvé : | 65,3 | 6,7 | 9,6 |

Préparation du 4-/N-(1H-indol-4-yl) amido/phénol.

On ajoute lentement sous atmosphère inerte 42 cm³ de tri-isobutylaluminium en solution toluénique (1,1M) à une solution de 3 g de 4-amino indole dans 100 cm³ de chloroforme. On ajoute ensuite 3,5 g de parahydroxybenzoate de méthyle dans 50 cm³ de chloroforme puis chauffe au reflux pendant 24 heures. On refroidit, ajoute 200 cm³ d'acide chlorhydrique 2N, agite pendant 30 minutes, filtre le précipité, le lave à l'eau et le sèche sous pression réduite à 80°C. On obtient 4,9 g de produit brut attendu.

>Spectre UV (éthanol)

| | | | |
|---|---|---|---|
| max. | 212 nm | $E^1_1$ = 1455 | $\varepsilon$= 36700 |
| max. | 256 nm | $E^1_1$ = 557 | $\varepsilon$= 14000 |
| max. | 277 nm | $E^1_1$ = 438 | $\varepsilon$= 11000 |
| max. | 284 nm | $E^1_1$ = 444 | $\varepsilon$= 11200 |
| infl. | 294 nm | $E^1_1$ = 432. | |

**Example N° 24 : 4-chloro 2-/3-/(1,1-diméthyléthyl) amino/2-hydroxy propoxy/N-(1H-indol 4-yl) benzamide et son oxalate neutre.**

**Stade A** : 4-chloro 2-/(2-oxiranyl) méthoxy/N-(1H-indol-4-yl) benzamide.

On chauffe au reflux pendant 24 heures sous atmosphère inerte 4,5 g de 5-chloro 2-/N-(1H-indol-4-yl) amino phénol dans 150 cm³ d'acétone avec 2,2 g de carbonate de potassium et 12,5 cm³ d'épichlorhydrine. Le produit cristallise dans le milieu réactionnel ; on élimine le solvant sous pression réduite à 50°C, reprend le résidu à l'eau, le filtre, le sèche sous pression réduite à 80°C. On obtient 5 g de produit attendu. F=194°C.

**Stade B** : 4-chloro 2-/3-/(1,1-diméthyléthyl) amino/2-hydroxy propoxy/N-(1H-indol-4-yl) benzamide et son oxalate neutre.

On chauffe 2 heures au reflux 5 g du produit précédent dans 100 cm³ d'éthanol et 7,6 cm³ de terbutylamine puis chasse le solvant sous pression réduite à 50°C. Après chromatographie sur silice (éluant : chloroforme-méthanol-triéthylamine 8-1-1), on recueille 5 g de produit attendu sous forme de base.

Formation de l'oxalate.

On dissout 5 g de la base ci-dessus dans 300 cm³ d'isopropanol et 300 cm³ de méthanol au reflux, puis ajoute 1,5 g d'acide oxalique et maintient le chauffage pendant 15 minutes. On concentre partiellement, glace, filtre et sèche sous pression réduite à 80°C le produit cristallisé. On obtient 4,4 g de produit attendu. F=254°C.

Analyse : $C_{22}H_{26}N_3ClO_3$, 1/2 $C_2H_2O_4$ : 460,941.

| | | | | |
|---|---|---|---|---|
| Calculé : | C% 59,93 | H% 5,90 | N% 9,12 | Cl% 7,69 |
| Trouvé : | 59,9 | 6,1 | 8,9 | 7,6 |

Préparation du 5-chloro 2-N-(1H-indol-4-yl) amido/phénol.

On chauffe au reflux pendant 2 heures sous atmosphère inerte sous atmosphère inerte la solution comprenant 5,28 g de 4-amino indole, 100 cm³ de tétrahydrofuranne, 6,9 g d'acide 4-chlorosalicylique et 9,06 g de dicyclohexylcarbodiimide. On filtre le dicyclohexylurée formée et chasse le solvant sous pression réduite à 50°C. Après chromatographie sur silice (éluant : chloroforme-acétate d'éthyle (9-1), on empâte le résidu à l'éther, sèche et obtient 5,55 g de produit attendu. F=245°C.

Analyse : $C_{15}H_{11}N_2ClO_2$ : 286,720.

| | | | | |
|---|---|---|---|---|
| Calculé : | C% 62,84 | H% 3,87 | N% 9,77 | Cl% 12,36 |
| Trouvé : | 62,5 | 4,0 | 9,7 | 12,3 |

**Exemple N° 25 : 2-/3-/(1,1-diméthyléthyl) amino/2-hydroxy propoxy/N-(1H-indol-4-yl) 5-méthoxy benzamide et son oxalate neutre.**

**Stade A** : 5-méthoxy 2-/(2-oxiranyl) méthoxy/N-(1H-ndol-4-yl) benzamide.

On chauffe au reflux pendant 20 heures sous atmosphère inerte 4,5 g de 4-méthoxy 2-/N-(1H-indol-4-yl) amido/phénol dans 150 cm³ d'acétone avec 2,2 g de carbonate de potassium et 12,5 cm³ d'épichlorhydrine. On filtre le carbonate de potassium, chasse le solvant sous pression réduite à 50°C et chromatographie le résidu sur silice (éluant : chloroforme-acétate d'éthyle 9-1). On recueille 5,4 g de produit que l'on empâte dans l'éther, filtre et sèche sous pression réduite. On obtient 5 g de produit attendu. F=122°C.

**Stade B** : 2-/3-/(1,1-diméthyléthyl) amino/2-hydroxy propoxy/N-(1H-indol-4-yl) 5-méthoxy benzamide et son oxalate neutre.

On opère comme au stade B de l'exemple 24 en utilisant au départ 5 g du produit du stade A ci-dessus et obtient 4,9 g de produit attendu sous forme de base.

Formation de l'oxalate neutre.

On opère comme au stade B de l'exemple 24 à partir de 4,9 g de la base obtenue ci-dessus et obtient 3,2 g de produit attendu. F=254°C.

Analyse : $C_{23}H_{29}N_3O_4$, 1/2 $C_2H_2O_4$ : 456,523.

| | | | |
|---|---|---|---|
| Calculé : | C% 63,14 | H% 6,62 | N% 9,20 |
| Trouvé : | 62,9 | 6,7 | 9,0 |

Préparation du 4-méthoxy 2-/N-(1H-indol-4-yl) amido/phénol.

On chauffe 6 heures au reflux sous atmosphère inerte 3,96 g de 4-amino indole dans 70 $cm^3$ de tétrahydrofuranne avec 5 g d'acide 5-méthoxy salicylique et 6,18 g de dicyclohexylcarbodiimide. On ajoute de nouveau 618 mg de dicyclohexylcarbodiimide et poursuit l'agitation à température ambiante pendant 20 heures. On filtre, élimine le solvant sous pression réduite à 50°C, reprend le résidu par 300 $cm^3$ d'acétate d'éthyle, lave la phase organique avec de l'acide chlorhydrique 2N, puis avec une solution aqueuse saturée en chlorure de sodium, sèche et élimine le solvant sous pression réduite à 50°C. On obtient 9,4 g de produit brut que l'on purifie par chromatographie sur silice (éluant : chloroforme-acétate d'éthyle 9-1). On empâte le produit recueilli dans l'éther isopropylique, filtre, sèche et obtient 4,7 g de produit attendu. F=225°C.

**Exemple N° 26 : 2-/3-(cyclohexylméthyl) amino/2-hydroxy propoxy/N-(1Hindol-4-yl) benzamide.**

On dissout en chauffant au reflux 2,5 g de 2-/(2-oxiranyl) méthoxy/N-(1H-indol-4-yl) benzamide préparé comme à l'exemple 4 dans 50 $cm^3$ d'éthanol en présence de 1,147 g de cyclohexane méthylamine et poursuit le chauffage pendant 2 heures et 15 minutes. On élimine le solvant sous pression réduite, chromatographie le résidu sur silice (éluant : chlorure de méthylène-méthanol 9-1) et obtient 1,97 g de produit attendu.

Spectre UV (éthanol)

| | | | |
|---|---|---|---|
| infl. | 215 nm | $E^1_1$ = | 385 |
| infl. | 234 nm | $E^1_1$ = | 365 |
| infl. | 269 nm | $E^1_1$ = | 128 |
| infl. | 292 nm | $E^1_1$ = | 229 |
| max. | 297 nm | $E^1_1$ = | 237 $\varepsilon$ = 10000 |
| infl. | 310 nm | $E^1_1$ = | 211. |

**Exemple N° 27 : 2-/3-//2-(3,4-diméthoxyphényl) éthyl/méthylamino/2-hydroxy propoxy/N-(1H-indol-4-yl) benzamide.**

On dissout à température ambiante sous atmosphère inerte 0,2 g de 2/3-//2-(3,4-diméthoxyphényl) éthyl/amino/2-hydroxy propoxy/N-(1H-indol-4-yl) benzamide préparé comme à l'exemple 21 dans 5 $cm^3$ de méthanol en présence de 0,1 $cm^3$ de formaldéhyde à 40% d'eau. On ajoute une solution comprenant 26 mg de cyanoborohydrure de sodium et 27,2 mg de chlorure de zinc dans 5 $cm^3$ de méthanol et abandonne 2 heures à température ambiante. On ajoute 5 $cm^3$ de soude 0,1N, extrait à l'acétate d'éthyle, lave la phase organique à l'eau, la sèche et élimine les solvants sous pression réduite. On recueille 0,230 g de produit brut que l'on purifie par chromatographie sur silice (éluant : chloroforme-acétate d'éthyle-triéthylamine 6-3-1). On obtient 0,150 g de produit attendu.

Spectre UV (éthanol)

| | | | | | |
|---|---|---|---|---|---|
| infl. | 218 nm | $E^1_1$ = | 794 | | |
| infl. | 230 nm | $E^1_1$ = | 429 | | |
| max. | 286 nm | $E^1_1$ = | 181 | | |
| max. | 298 nm | $E^1_1$ = | 176 | $\varepsilon$ = | 8850 |
| infl. | 310 nm | $E^1_1$ = | 158 | | |
| infl. | 318 nm | $E^1_1$ = | 147. | | |

## Etude pharmacologique

1) Action antiarythmique chez le rat

On trachéotomise des rats mâles pesant 300-350 g anesthésiés par voie intrapéritonéale à l'aide de 1,20 g/kg d'uréthane et les soumet à une respiration artificielle (40-50 insufflations de 3 ml/minute).

On implante des aiguilles en sous cutané de manière à enregistrer l'électrocardiogramme des rats sur le signal en dérivation DII.

On administre les produits à tester par voie intraveineuse ou par voie orale.

Cinq minutes après l'administration du produit par voie intraveineuse ou 1 heure après administration par voie orale, on perfuse la veine jugulaire des rats avec 10 µg/mn d'une solution d'aconitine et on note le temps d'apparition des troubles du rythme cardiaque (10 µg d'aconitine correspondant à la perfusion de 0,2 ml de solution.)

Les résultats sont exprimés en pourcentage d'allongement du temps d'apparition des troubles du rythme cardiaque par rapport aux témoins et en fonction de la dose du produit testé. Les résultats figurant sur le tableau ci-après montrent que les produits de la présente demande sont doués de remarquables propriétés antiarythmiques.

| Produit de l'exemple | voie | Dose en mg/kg | Pourcentage d'allongement du temps |
|---|---|---|---|
| 1 | IV | 0,25 | + 31 |
| | | 0,5 | + 41 |
| | | 1 | + 110 |
| | | | |
| 2 | IV | 1 | + 6 |
| | | 2,5 | + 57 |
| | | | |
| 3 | IV | 2,5 | + 68 |
| | | | |
| 4 | IV | 0,5 | + 17 |
| | | 1 | + 38 |
| | | 2,5 | + 124 |
| | | | |
| | PO | 5 | + 7 |
| | | 10 | + 46 |
| | | 25 | + 81 |
| | | | |
| 5 | IV | 0,25 | + 9 |
| | | 0,5 | + 36 |
| | | 1 | + 33 |
| | | 2,5 | + 61 |

| Produit de l'exemple | voie | Dose en mg/kg | Pourcentage d'allongement du temps |
|---|---|---|---|
| | PO | 2,5 | + 22 |
| | | 5 | + 32 |
| | | 10 | + 59 |
| 6 | IV | 1 | + 23 |
| | | 2,5 | + 41 |
| | PO | 25 | + 29 |
| 15 | IV | 2,5 | + 25,5 |
| | | 5 | + 56 |

2) Etude de la toxicité aigüe

On a évalué les doses létales $DL_0$ des différents composés testés après administration par voie orale chez la souris.

On appelle $DL_0$ la dose maximale ne provoquant aucune mortalité en 8 jours.

Les résultats obtenus sont les suivants :

| Produit de l'exemple | $DL_0$ en mg/kg |
|---|---|
| 1 | 80 |
| 2 | 80 |
| 4 | > 400 |
| 5 | 60 |
| 6 | 200 |
| 12 | > 400 |
| 15 | $\geqslant$ 400 |

## Revendications

1. Les dérivés de l'indole carboxamide ainsi que leurs sels d'addition avec les acides minéraux ou organiques, caractérisés en ce qu'ils répondent à la formule générale (I) :

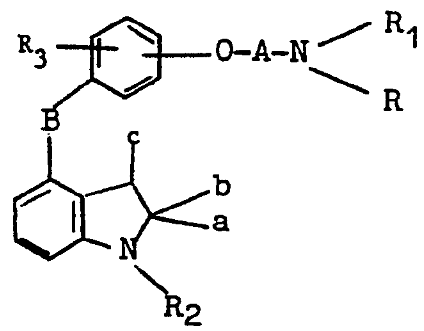

I

dans laquelle R et $R_1$ représentent, indépendamment l'un de l'autre, un atome d'hydrogène, un radical alkyle linéaire renfermant de 1 à 5 atomes de carbone, un radical alkyle ramifié renfermant de 3 à 5 atomes de carbone, un radical cycloalkyle renfermant de 3 à 7 atomes de carbone, un radical cycloalkylalkyle renfermant de 4 à 7 atomes de carbone, ou un radical aralkyle renfermant de 7 à 12 atomes de carbone éventuellement substitué par 1, 2 ou 3 radicaux choisis dans le groupe constitué par les halogènes et les radicaux méthyle, éthyle, méthoxy, éthoxy, trifluorométhyle, méthylthio, amino et nitro, ou R et $R_1$ forment ensemble un hétérocycle saturé ou insaturé pouvant renfermer un second hétéroatome choisi parmi les atomes d'oxygène, de soufre et d'azote, cet atome d'azote étant éventuellement substitué par un radical alkyle renfermant de 1 à 5 atomes de carbone, par un radical phényle, phényle substitué ou naphtyle ou par un radical aralkyle renfermant de 7 à 12 atomes de carbone, $R_3$ représente un atome d'hydrogène, un radical alkyle renfermant de 1 à 5 atomes de carbone, un radical alkoxy renfermant de 1 à 3 atomes de carbone, un atome de chlore, de brome ou d'iode, un radical nitro ou un radical amino éventuellement substitué par un groupe acyle aliphatique renfermant de 2 à 5 atomes de carbone ou par un radical alkyle renfermant de 1 à 5 atomes de carbone, a représente ensemble avec b une fonction oxo, ou représente ensemble avec c une liaison carbone-carbone, b représente un atome d'hydrogène, ou ensemble avec a une fonction oxo, c représente un atome d'hydrogène, ou ensemble avec a représente une liaison carbone-carbone, A représente une chaîne $-(CH_2)_n-$ dans laquelle n peut prendre les valeurs 2, 3, 4 ou 5, ou une chaîne

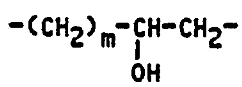

dans laquelle m peut prendre les valeurs 1, 2 ou 3, B représente une chaîne –CO–NH– ou –NH–CO–, $R_2$ représente un atome d'hydrogène, un radical alkyle linéaire renfermant de 1 à 5 atomes de carbone ou un radical alkyle ramifié renfermant de 3 à 5 atomes de carbone.

2. Les dérivés de l'indole carboxamide tels que définis par la formule I de la revendication 1, ainsi que leurs sels d'addition avec les acides minéraux ou organiques, caractérisés en ce que $R_2$ représente un atome d'hydrogène.

3. Les dérivés de l'indole carboxamide selon la revendication 2, ainsi que leurs sels d'addition avec les acides minéraux ou organiques, caractérisés en ce que a et c forment ensemble une liaison carbone-carbone.

4. Les dérivés de l'indole carboxamide selon l'une des revendications 1 à 3 ainsi que leurs sels d'addition avec les acides minéraux ou organiques, caractérisés en ce que $R_3$ représente un atome d'hydrogène.

5. Les dérivés de l'indole carboxamide selon l'une des revendications 1 à 4 ainsi que leurs sels d'addition avec les acides minéraux ou organiques, caractérisés en ce que la chaîne latérale

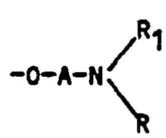

est fixée en position ortho.

6. Les dérivés de l'indole carboxamide selon l'une des revendications 1 à 5 ainsi que leurs sels d'addition avec les acides minéraux ou organiques, caractérisés en ce que B représente une chaîne –NH–CO, NH étant du côté de l'indole.

7. Les dérivés de formule (I) selon la revendication 1 dont les noms suivent :
- le 2-/2-/(1,1-diméthyléthyl) amino/éthoxy/N-(1H-indol-4-yl) benzamide,
- le 2-/3-/(1,1-diméthyléthyl) amino/2-hydroxy propoxy/N-(1H-indol-4-yl) benzamide, ainsi que leurs sels d'addition avec les acides minéraux ou organiques.

8. Les dérivés de formule (I) selon la revendication 1 dont les noms suivent :
- le 2-/3-//bis-(1-méthyléthyl)/amino/2-hydroxypropoxy/N-(1H-indol-4-yl) benzamide,
- le 2-/3-/(1,1-diméthyléthyl) amino/propoxy/N-(1H-indol-4-yl) benzamide, ainsi que par leurs sels d'addition avec les acides minéraux ou organiques.

9. Procédé de préparation des dérivés tels que définis par la formule I de la revendication 1, ainsi que de leurs sels, caractérisé en ce que l'on fait réagir un dérivé de formule II :

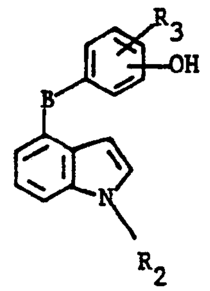

II

dans laquelle B, $R_2$ et $R_3$ ont la signification déjà indiquée, avec un halogénure de formule III :

Hal-G III

dans laquelle Hal représente un atome de chlore, de brome ou d'iode et G représente un groupement de formule $-(CH_2)_n-D$, dans laquelle D représente un atome de chlore, de brome, d'iode, un radical hydroxy ou un sulfonate de ce radical hydroxy, et n a la signification déjà indiquée, ou G représente un groupement

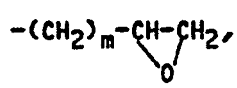

dans laquelle m a la signification déjà indiquée, pour obtenir un dérivé de formule IV :

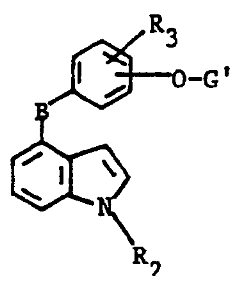

IV

dans laquelle B, $R_2$ et $R_3$ ont la signification déjà indiquée, et G' représente un groupement de formule $-(CH_2)_n-$Hal dans laquelle n et Hal ont la signification déjà indiquée ou un groupement de formule

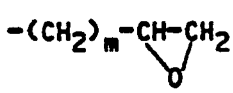

défini ci-dessus, que l'on fait réagir avec une amine de formule V :

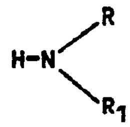

V

dans laquelle R et $R_1$ ont la signification déjà indiquée, pour obtenir un produit de formule $I_A$ :

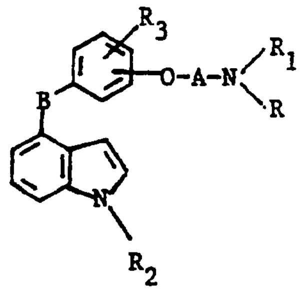

$I_A$

dans laquelle A, B, R, $R_1$, $R_2$ et $R_3$ ont la signification déjà indiquée, que soit l'on isole et, si désiré, salifie, ou si désiré, soumet à un agent d'alkylation, l'amine secondaire de la chaîne latérale lorsque R ou $R_1$ représente un atome d'hydrogène, soit soumet à l'action d'un agent d'halogénation pour obtenir un produit de formule VI :

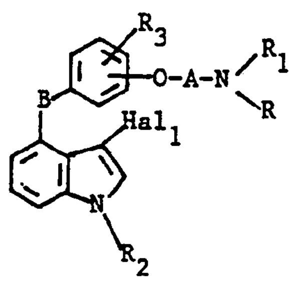

VI

dans laquelle $Hal_1$ représente un atome de brome ou de chlore, et A, B, R, $R_1$, $R_2$ et $R_3$ ont la signification déjà indiquée, que l'on soumet à une hydrolyse pour obtenir un produit de formule $I_B$ :

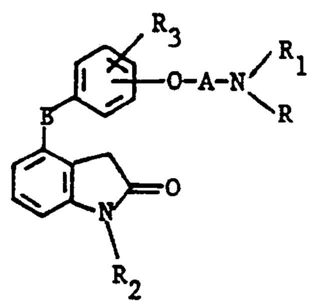

$I_B$

dans laquelle A, B, R, $R_1$, $R_2$ et $R_3$ ont la signification déjà indiquée, que l'on isole et, si désiré, salifie.

10. Procédé selon la revendication 9, caractérisé en ce que la réaction du dérivé de formule II avec l'halogénure de formule III est réalisée :

– en présence d'une base lorsque G représente une chaîne

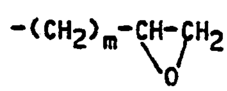

– avec le sulfonate de formule Ts–O–$(CH_2)_n$Hal dans laquelle Ts représente un radical tosyle et Hal a la signification déjà indiquée lorsque G représente une chaîne $-(CH_2)_n-b$.

11. Variante du procédé selon la revendication 9 pour la préparation des dérivés de formule I dans laquelle A représente une chaîne $-(CH_2)_n$, caractérisée en ce que l'on fait réagir un dérivé de formule II avec un dérivé

de formule IX :

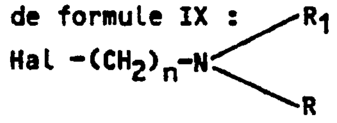

dans laquelle Hal, n, R et $R_1$ ont la signification déjà indiquée, pour obtenir le dérivé de formule $I_A$ correspondant que l'on transforme si désiré en produit de formule $I_B$ correspondant.

12. Médicaments, caractérisés en ce qu'ils sont constitués par les dérivés de l'indole carboxamide, tels que définis par la formule I de la revendication 1, ainsi que par leurs sels d'addition avec les acides pharmaceutiquement acceptables.

13. Médicaments, caractérisés en ce qu'ils sont constitués par les dérivés de l'indole carboxamide, tels que définis dans l'une des revendications 2 ou 3, ainsi que par leurs sels d'addition avec les acides pharmaceutiquement acceptables.

14. Médicaments, caractérisés en ce qu'ils sont constitués mar les dérivés de l'indole carboxamide tels que définis dans l'une des revendications 4, 5 ou 6 ainsi que par leurs sels d'addition avec les acides pharmaceutiquement acceptables.

15. Médicaments, caractérisés en ce qu'ils sont constitués par lesdérivés de l'indole carboxamide, tels que définis à la revendication 7 ou 8, ainsi que par leurs sels d'addition avec les acides pharmaceutiquement acceptables.

16. Compositions pharmaceutiques, caractérisées en ce qu'elles renferment à titre de principe actif, l'un au moins des médicaments, tels que définis à l'une des revendications 12 à 15.

17. Les dérivés choisis parmi les formules II, IV et VI suivantes :

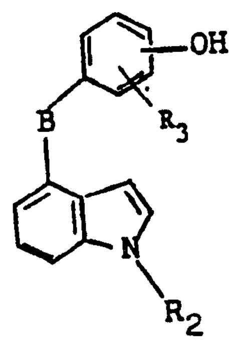

II

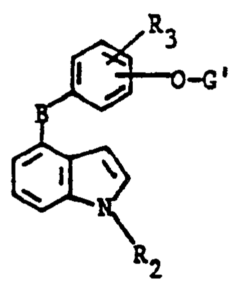

IV

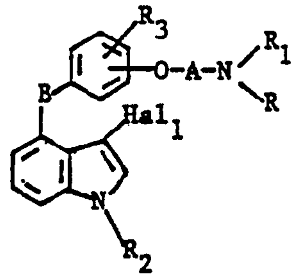

VI

formules dans lesquelles B, R, $R_1$, $R_2$ et $R_3$ ont la signification indiquée à la revendication I et G′ et $Hal_1$ ont la signification indiquée à la revendication 9.

**Ansprüche**

1. Indolcarboxamidderivate sowie deren Additionssalze mit Mineral- oder organischen Säuren, dadurch gekennzeichnet, daß sie der allgemeinen Formel (I)

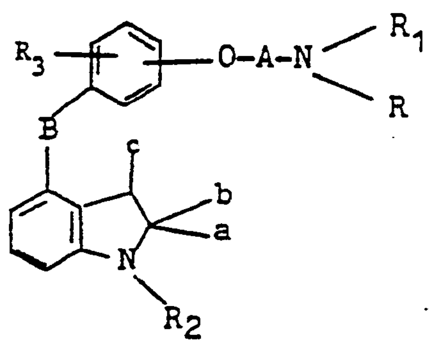

(I)

entsprechen, worin R und $R_1$ unabhängig voneinander ein Wasserstoffatom, eine lineare Alkylgruppe mit 1 bis 5 Kohlenstoffatomen, eine verzweigte Alkylgruppe mit 3 bis 5 Kohlenstoffatomen, eine Cycloalkylgruppe mit 3 bis 7 Kohlenstoffatomen, eine Cycloalkylalkylgruppe mit 4 bis 7 Kohlenstoffatomen oder eine Aralkylgruppe mit 7 bis 12 Kohlenstoffatomen, gegebenenfalls substituiert durch 1, 2 oder 3 Reste, ausgewählt unter den Halogenen, den Methyl-, Ethyl-, Methoxy-, Ethoxy-, Trifluormethyl-, Methylthio-, Amino- und Nitrogruppen, bedeuten oder R und $R_1$ gemeinsam einen gesättigten oder ungesättigten Heterocyclus, der ein zweites Heteroatom, ausgewählt unten den Sauerstoff-, Schwefel- und Stickstoffatomen, enthalten kann, bilden, wobei dieses Stickstoffatom gegebenenfalls substituiert ist durch eine Alkylgruppe mit 1 bis 5 Kohlenstoffatomen, durch eine Phenylgruppe, eine substituierte Phenylgruppe oder Naphthylgruppe oder durch eine Aralkylgruppe mit 7 bis 12 Kohlenstoffatomen, $R_3$ für ein Wasserstoffatom, eine Alkylgruppe mit 1 bis 5 Kohlenstoffatomen, eine Alkoxygruppe mit 1 bis 3 Kohlenstoffatomen, ein Chlor-, Brom- oder Jodatom, eine Nitrogruppe oder eine Aminogruppe, gegebenenfalls substituiert durch eine aliphatische Acylgruppe mit 2 bis 5 Kohlenstoffatomen oder durch eine Alkylgruppe mit 1 bis 5 Kohlenstoffatomen, steht, a gemeinsam mit b eine Oxofunktion wiedergibt oder gemeinsam mit c eine Kohlenstoff-Kohlenstoff-Bindung wiedergibt, b ein Wasserstoffatom bedeutet oder gemeinsam mit a eine Oxofunktion bildet, c ein Wasserstoffatom bedeutet oder gemeinsam mit a eine Kohlenstoff-Kohlenstoff-Bindung wiedergibt, A eine Kette $-(CH_2)_n-$ bedeutet, worin n die Werte 2, 3, 4 oder 5 annehmen kann, oder eine Kette

$$-(CH_2)_m-\underset{OH}{CH}-CH_2-$$

bedeutet, worin m die Werte 1, 2 oder 3 annehmen kann, B für eine Kette –CO–NH– oder –NH–CO– steht, $R_2$ ein Wasserstoffatom, eine lineare Alkylgruppe mit 1 bis 5 Kohlenstoffatomen oder eine verzweigte Alkylgruppe mit 3 bis 5 Kohlenstoffatomen wiedergibt.

2. Indolcarboxamidderivate der formel I gemäß Anspruch 1 sowie deren Additionssalze mit Mineral-

oder organischen Säuren, dadurch gekennzeichnet, daß $R_2$ ein Wasserstoffatom bedeutet.

3. Indolcarboxamidderivate gemäß Anspruch 2 sowie deren Additionssalze mit Mineral- oder organischen Säuren, dadurch gekennzeichnet, daß a und c gemeinsam eine Kohlenstoff-Kohlenstoff-Bindung bilden.

4. Indolcarboxamidderivate gemäß einem der Ansprüche 1 bis 3 sowie deren Additionssalze mit Mineral- oder organischen Säuren, dadurch gekennzeichnet, daß $R_3$ ein Wasserstoffatom bedeutet.

5. Indolcarboxamidderivate gemäß einem der Ansprüche 1 bis 4 sowie deren Additionssalze mit Mineral- oder organischen Säuren, dadurch gekennzeichnet, daß die Seitenkette

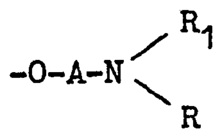

in ortho-Stellung gebunden ist.

6. Indolcarboxamidderivate gemäß einem der Ansprüche 1 bis 5 sowie deren Additionssalze mit Mineral- oder organischen Säuren, dadurch gekennzeichnet, daß B für eine Kette –NH–CO steht, wobei NH an der Seite des Indols liegt.

7. Die Derivate der formel (I) gemäß Anspruch 1 mit den folgenden Bezeichnungen :

2-[2-[(1,1-Dimethylethyl)-amino]-ethoxy]-N-(1H-indol-4-yl)-benzamid,

2-[3-[(1,1-Dimethylethyl)-amino]-2-hydroxypropoxy]-N-(1H-indol-4-yl)-benzamid sowie deren Additionssalze mit Mineral- oder organischen Säuren.

8. Die Derivate der formel (I) gemäß Anspruch 1 mit den folgenden Bezeichnungen :

2-[3-[[Bis-(1-methylethyl)]-amino]-2-hydroxypropoxy]-N-(1H-indol-4-yl)-benzamid,

2-[3-[(1,1-Dimethylethyl)-amino]-propoxy]-N-(1H-indol-4-yl)-benzamid sowie deren Additionssalze mit Mineral- oder organischen Säuren.

9. Verfahren zur Herstellung der Derivate der formel I gemäß Anspruch 1 sowie von deren Salzen, dadurch gekennzeichnet, daß man ein Derivat der formel II

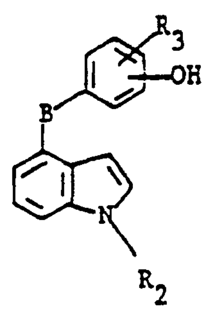

(II)

worin B, $R_2$ und $R_3$ die angegebene Bedeutung besitzen, mit einem Halogenid der formel III

Hal-G (III)

worin Hal für ein Chlor-, Brom- oder Jodatom steht und G eine Gruppe der formel $-(CH_2)_n-D$ wiedergibt, in der D ein Chlor-, Brom-, Jodatom, eine Hydroxygruppe oder ein Sulfonat dieses Hydroxyrestes bedeutet und n die angegebene Bedeutung besitzt, oder G für eine Gruppe

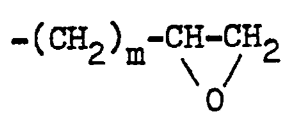

steht, worin m die angegebene Bedeutung besitzt, umsetzt, um zu einem Derivat der formel IV

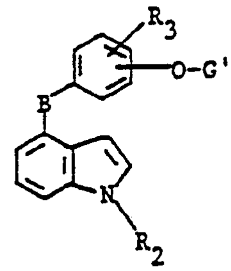

(IV)

zu gelangen, worin B, $R_2$ und $R_3$ die angegebene Bedeutung besitzen und G′ für eine Gruppe der formel $-(CH_2)_n-$Hal steht, in der n und Hal die angegebene Bedeutung haben, oder eine Gruppe der formel

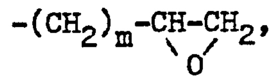

wie vorstehend definiert, steht, welches man mit einem Amin der formel V

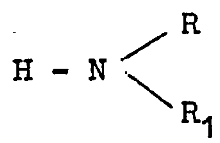

(V)

worin R und $R_1$ die angegebene Bedeutung besitzen, umsetzt, um zu einem Produkt der formel $I_A$

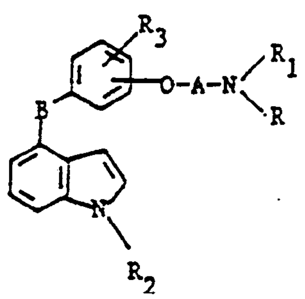

($I_A$)

zu gelangen, worin A, B, R, $R_1$, $R_2$ und $R_3$ die angegebene Bedeutung besitzen, welches man entweder isoliert und gewünschtenfalls in ein Salz überführt oder gewünschtenfalls das sekundäre Amin der Seitenkette der Einwirkung eines Alkylierungsmittels unterzieht, wenn R oder $R_1$ ein Wasserstoffatom bedeuten, oder der Einwirkung eines Halogenierungsmittels unterzieht, um zu einem Produkt der formel VI

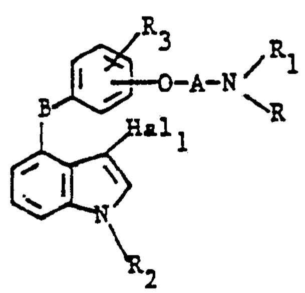

(VI)

zu gelangen, worin $Hal_1$ für ein Brom- oder Chloratom steht und A, B, R, $R_1$, $R_2$ und $R_3$ die angegebene Bedeutung besitzen, welches man einer Hydrolyse unterzieht, um zu einem Produkt der formel $I_B$

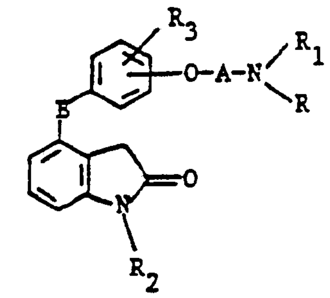

($I_B$)

zu gelangen, worin A, B, R, $R_1$, $R_2$ und $R_3$ die angegebene Bedeutung besitzen, welches man isoliert und gewünschtenfalls in ein Salz überführt.

10. Verfahren gemäß Anspruch 9, dadurch gekennzeichnet, daß die Umsetzung des Derivats der formel II mit dem Halogenid der formel III durchgeführt wird

– in Gegenwart einer Base, wenn G eine Kette

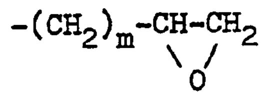

bedeutet,

– mit dem Sulfonat der formel $Ts-O-(CH_2)_nHal$, worin Ts für einen Tosylrest steht und Hal die angegebene Bedeutung besitzt, wenn G eine Kette $-(CH_2)_n-b$ wiedergibt.

– 11. Abänderung des Verfahrens gemäß Anspruch 9 zur Herstellung der Derivate der formel I, worin A eine Kette $-(CH_2)_n$ wiedergibt, dadurch gekennzeichnet, daß man ein Derivat der formel II mit einem Derivat der formel IX

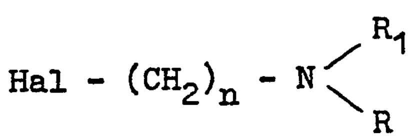

worin Hal, n, R und $R_1$ die angegebene Bedeutung besitzen, umsetzt, um zu dem entsprechenden Derivat der formel $I_A$ zu gelangen, das man gewünschtenfalls in das entsprechende Produkt der formel $I_B$ überführt.

12. Arzneimittel, dadurch gekennzeichnet, daß sie aus den Indolcarboxamidderivaten der formel I gemäß Anspruch 1 oder aus deren Additionssalzen mit pharmazeutisch verträglichen Säuren bestehen.

13. Arzneimittel, dadurch gekennzeichnet, daß sie aus den Indolcarboxamidderivaten gemäß einem der Ansprüche 2 oder 3 sowie aus deren Additionssalzen mit pharmazeutisch verträglichen Säuren bestehen.

14. Arzneimittel, dadurch gekennzeichnet, daß sie aus den Indolcarboxamidderivaten gemäß einem der Ansprüche 4, 5 oder 6 sowie aus deren Additionssalzen mit pharmazeutisch verträglichen Säuren bestehen.

15. Arzneimittel, dadurch gekennzeichnet, daß sie aus den Indolcarboxamidderivaten gemäß Anspruch 7 oder 8 sowie aus deren Additionssalzen mit pharmazeutisch verträglichen Säuren bestehen.

16. Pharmazeutische Zusammensetzungen, dadurch gekennzeichnet, daß sie als Wirkstoff zumindest eines der Arzneimittel gemäß einem der Ansprüche 12 bis 15 enthalten.

17. Die Derivate, ausgewählt unter den folgenden formeln II, IV und VI

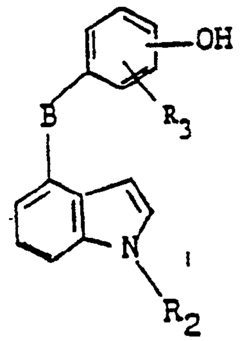

II

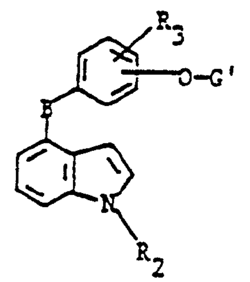

IV

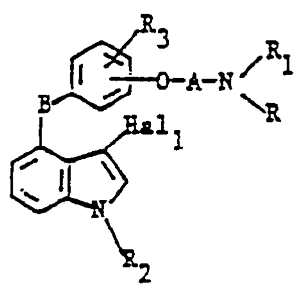

VI

worin B, R, $R_1$, $R_2$ und $R_3$ die in Anspruch 1 angegebene Bedeutung besitzen und G′ und $Hal_1$ die in Anspruch 9 angegebene Bedeutung haben.

**Claims**

1. The derivatives of indole carboxamide as well as their addition salts with mineral or organic acids, characterized in that they correspond to the general formula (I) :

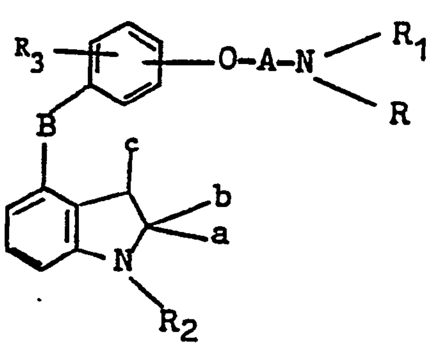

(I)

in which R and $R_1$ represent, independently of each other, a hydrogen atom, a linear alkyl radical containing 1 to 5 carbon atoms, a branched alkyl radical containing 3 to 5 carbon atoms, a cycloalkyl radical containing 3 to 7 carbon atoms, a cycloalkylalkyl radical containing 4 to 7 carbon atoms, or an aralkyl radical containing 7 to 12 carbon atoms optionally substituted by 1, 2 or 3 radicals chosen from the group constituted by halogens and methyl, ethyl, methoxy,ethoxy, trifluoromethyl, methylthio, amino and nitro radicals, or R and $R_1$ together form a saturated or unsaturated heterocycle which can contain a second heteroatom chosen from oxygen, sulphur and nitrogen atoms, this nitrogen atom being optionally substituted by an alkyl radical containing 1 to 5 carbon atoms, by a phenyl, substituted phenyl or naphthyl radical or by an aralkyl radical containing 7 to 12 carbon atoms, R3 represents a hydrogen atom, an alkyl radical containing 1 to 5 carbon atoms, an alkoxy radical containing 1 to 3 carbon atoms, a chlorine, bromine or iodine atom, a nitro radical or an amino radical optionally substituted by an aliphatic acyl group containing 2 to 5 carbon atoms or by an alkyl group containing 1 to 5 carbon atoms, a, together with b, represents an oxo function, or together with c represents a carbon-carbon bond, b represents a hydrogen atom, or together with a, an oxo function, c represents a hydrogen atom, or together with a, a carbon-carbon bond, A represents a $-(CH_2)_n-$ chain in which n can have the values 2, 3, 4 or 5, or a

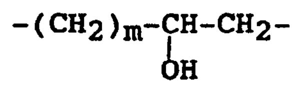

chain in which m can have the values 1, 2 or 3, B represents a –CO–NH– or –NH–CO– chain, $R_2$ represents a hydrogen atom, a linear alkyl radical containing 1 to 5 carbon atoms or a branched alkyl radical containing 3 to 5 carbon atoms.

2. The derivatives of indole carboxamide as defined by formula (I) of claim 1, as well as their addition salts with mineral or organic acids, characterized in that R2 represents a hydrogen atom.

3. The derivatives of indole carboxamide according to claim 2, as well as their addition salts with mineral or organic acids, characterized in that a and c together form a carbon-carbon bond.

4. The derivatives of indole carboxamide according to one of claims 1 to 3 as well as their addition salts with mineral or organic acids, characterized in that R3 represents a hydrogen atom.

5. The derivatives of indole carboxamide according to one of claims 1 to 4, as well as their addition salts with mineral or organic acids, characterized in that the lateral chain

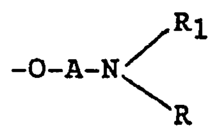

is fixed in ortho position.

6. The derivatives of indole carboxamide according to one of claims 1 to 5, as well as their addition salts with mineral or organic acids, characterized in that B represents an –NH–CO chain, NH being on the side of the indole.

7. The derivatives of formula (I) according to claim 1, of which the names follow :

– 2-[2-[(1,1-dimethylethyl)-amino]-ethoxy]-N-(1H-indol-4-yl)-benzamide,

– 2-[3-[(1,1-dimethylethyl)-amino]-2-hydroxypropoxy]-N-(1H-indo1-4-yl)-benzamide, as well as their addition salts with mineral or organic acids.

8. The derivatives of formula (I) according to claim 1, of which the names follow :

– 2-[3-[[bis-(1-methylethyl)]-amino]-2-hydroxypropoxy]-N-(1H-indol-4-yl)-benzamide,

– 2-[3-[(1,1-dimethylethyl)-amino]-propoxy]-N-(1H-indol-4-yl)-benzamide, as well as their addition salts with mineral or organic acids.

9. Process for the preparation of derivatives as defined by formula (I) of claim 1, as well as their salts, characterized in that a derivative of formula II :

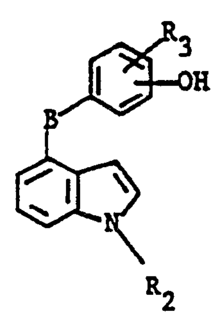

(II)

in which B, $R_2$ and $R_3$ have the meaning already indicated, is reacted with a halide of formula (III) :

Hal-G (III)

in which Hal represents a chlorine, bromine or iodine atom and G represents a group of formula $-(CH_2)_n-D$, in which D represents a chlorine, bromine or iodine atom, a hydroxy radical or a sulphonate of this hydroxy radical, and n has the meaning already indicated, or G represents a

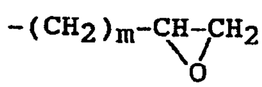

group, in which m has the meaning already indicated, so as to obtain a derivative of formula (IV) :

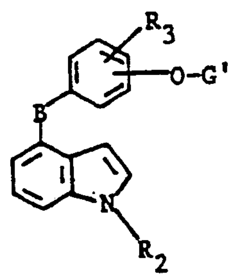

(IV)

in which B, $R_2$ and $R_3$ have the meaning already indicated, and G′ represents a group of formula $-(CH_2)_n-Hal$ in which n and Hal have the meaning already indicated, or a group of formula

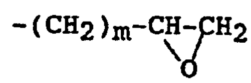

defined above, which is reacted with an amine of formula V :

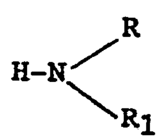

(V)

in which R and $R_1$ have the meaning already indicated, so as to obtain a product of formula ($I_A$) :

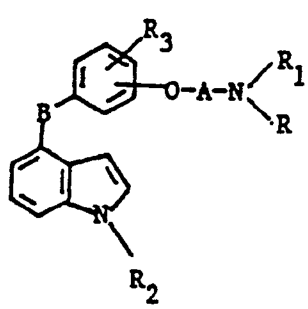

($I_A$)

in which A, B, R, $R_1$, $R_2$ and $R_3$ have the meaning already indicated, which either is isolated and, if desired, salified, or if desired, the secondary amine of the lateral chain is subjected to an alkylation agent when R or $R_1$ represents a hydrogen atom, or is subjected to the action of a halogenation agent so as to obtain a product of formula (VI) :

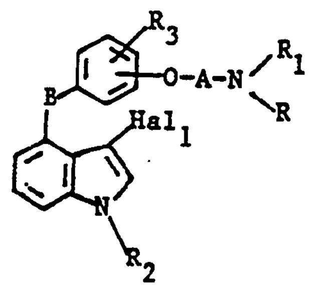

(VI)

in which $Hal_1$ represents a bromine or chlorine atom, and A, B, R, $R_1$, $R_2$ and $R_3$ have the meaning already indicated, which is subjected to a hydrolysis so as to obtain a product of formula ($I_B$) :

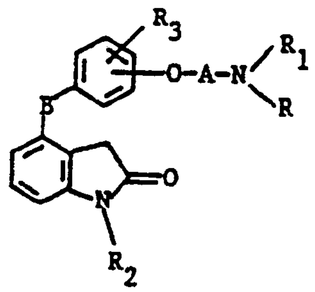

($I_B$)

in which A, B, R, $R_1$, $R_2$ and $R_3$ have the meaning already indicated, which is isolated, and, if desired, salified.

10. Process according to claim 9, characterized in that the reaction of the derivative of formula (II) with the halide of formula (III) is carried out :

– in the presence of a base when G represents a

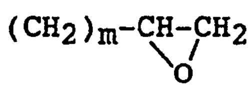

chain

– with the sulphonate of formula Ts–O–$(CH_2)_n$Hal in which Ts represents a tosyl radical and Hal has the meaning already indicated when G represents a $-(CH_2)_n-$b chain.

11. Variation of the process according to claim 9 for the preparation of derivatives of formula (I) in which A represents a $-(CH_2)_n$ chain, characterized in that a derivative of formula (II) is reacted with a derivative of formula (IX) :

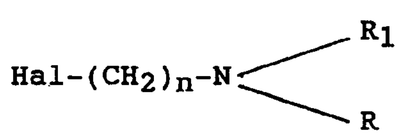

in which Hal, n, R and $R_1$ have the meaning already indicated, so as to obtain the corresponding derivative of formula ($I_A$) which is converted if desired into a corresponding product of formula ($I_B$).

12. Medicaments, characterized in that they are constituted by the derivatives of indole carboxamide, as defined by formula (I) of claim 1, as well as by their addition salts with pharmaceutically acceptable acids.

13. Medicaments, characterized in that they are constituted by the derivatives of indole carboxamide, as defined in one of claims 2 or 3, as well as by their addition salts with pharmaceutically acceptable acids.

14. Medicaments, characterized in that they are constituted by the derivatives of indole carboxamide as defined in one of claims 4, 5 or 6, as well as by their addition salts with pharmaceutically acceptable acids..

15. Medicaments, characterized in that they are constituted by the derivatives of indole carboxamide as defined in claim 7 or 8, as well as by their addition salts with pharmaceutically acceptable acids.

16. Pharmaceutical compositions, characterized in that they contain as active ingredient, at least one of the medicaments as defined in any one of claims 12 to 15.

17. The derivatives chosen from the following formulae (II), (IV) and (VI) :

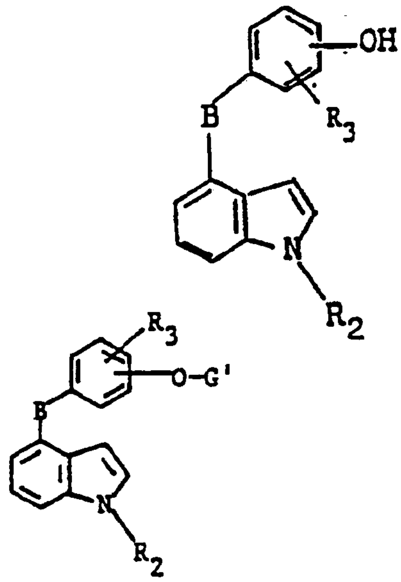

(II)

(IV)

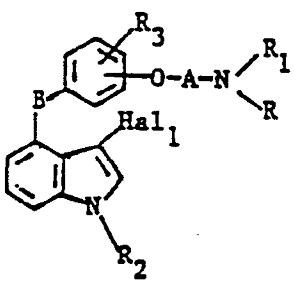

(VI)

in which formulae B, R, $R_1$, $R_2$ and $R_3$ have the meaning already indicated in claim 1 and G′ and $Hal_1$ have the meaning indicated in claim 9.